# EUROPEAN PATENT APPLICATION

(11) **EP 4 530 359 A1**
(43) Date of publication of application: **02.04.2025**
(21) Application number: 23200449.9
(22) Date of filing: 28.09.2023
(51) Int. Cl.: C12Q 1/6841

(54) **MULTIPLEXED SINGLE MOLECULE RNA FISH USING DNA NANOSTRUCTURES**

(71) Applicant: LMU Ludwig-Maximilians-Universität, 80539 München Bavaria (DE)
(72) Inventor: Port, Christina, 80796 München (DE)
(74) Representative: Stellbrink & Partner Patentanwälte mbB

(57) **Abstract**

The present invention relates to a probe for RNA fluorescence in situ hybridization (FISH) comprising: a marker molecule, a targeting molecule comprising 22 to 55 nt comprising a spacer sequence, and a target binding sequence capable of binding to a target molecule, wherein the marker molecule is connected to the targeting molecule via the spacer sequence. The present invention also relates to a composition for RNA FISH comprising a set of probes for RNA FISH, wherein the set of probes comprises at least 1 probe for RNA FISH, preferably at least 5 probes for RNA FISH, more preferably at least 10 probes for RNA FISH, wherein the set of probes comprises at least one probe. The invention further relates to a method for designing a probe for RNA FISH for analyzing a sample, wherein the method comprises providing a probe for RNA FISH, wherein the method comprises detecting at least one target molecule in situ in the sample using the probe. The present invention further relates to a kit for use in a molecular analysis, the comprising a probe according to any of the preceding probe claims, or a composition according to any of the preceding composition claims, and at least one buffer for hybridization comprising at least one of: fixation buffer component, wash buffer, and hybridization buffer.

## Description

### Field

The invention lies in the field of molecular biology and particularly in the field of RNA fluorescence in situ hybridization (RNA FISH) technology. The goal of the present invention is to provide a multiplexed single molecule RNA FISH using DNA nanotechnology and a method performed using such multiplexed single molecule RNA FISH.

### Introduction

Measurement of gene expression in individual cells in the spatial context of cells or tissue is becoming increasingly relevant not only to academic research, but also to industrial applications and medical molecular diagnostics. Bulk measurements of gene expression such as RT-qPCR, microarray technology, bulk RNA sequencing (RNASeq) averages abundance of RNA in all measured cells, which covers up individual differences in gene expression as well as obscures low-level gene expression of crucial genes. This approach results in relevant information being lost, for example, in the case with transcription factors such as GATA6, which in about 50% of cases is more lowly expressed than measurable via RT-qPCR or microarrays (PMID 32156747). Moreover, the corresponding spatial information of where the gene is expressed, i.e., where the transcripts are found in the cell, are lost in bulk measurements of gene expression.

This information, however, is critical on the tissue level (differential expression of genes in different cell types and tissue polarization) as well as the subcellular level (spatial compartmentalization of gene expression within cells, enrichment of RNAs at the endoplasmatic reticulum (ER), retainment in the nucleus (PMID 31501331)). These intricacies of gene expression are becoming increasingly relevant to academic research and its applications in molecular diagnostics.

In these areas, conventional RNA Fluorescence in Situ Hybridization (FISH), also called single molecule FISH, smFISH, is considered the gold standard for sensitivity in RNA in situ detection. However, the number of simultaneously measurable RNA species detectable by this method is limited by the number of clearly distinguishable fluorescence spectra which can be recorded simultaneously by a fluorescence microscope; usually 3-4 RNAs in one hybridization, greatly limiting the scalability of the method.

Alternative methods have been developed to overcome this limitation of either measuring a large number of RNA species, or obtaining spatial contextual information, for example a number of spatial sequencing technologies such as 10x Genomics' Visium, Readcoor's FISSEQ, gene expression platforms such as Nanostring GeoMx, and highly multiplexed RNA FISH methods such as merFISH, seqFISH.

EP 3 133 170 A1 relates to a probe set for in situ hybridization that enables detection of individual mRNA molecules, comprising at least 12 non-overlapping nucleic acid hybridization probes singly labelled with the same first fluorophore label of a first color, wherein the probes have sequences complementary to a target sequence, wherein said probes are 7-30 nucleotides in length.

EP 3 541 956 A1 relates to methods for spatial tagging of nucleic acid molecules in a biological specimen and in particular to a method comprising: providing a solid substrate on which multiple species of capture probes are immobilized; contacting a solid substrate with a biological specimen; and releasing capture probes from the surface of the solid substrate under conditions that allow nucleic acids of the biological specimen to hybridize.

EP 3 916 108 A1 relates to a method for spatially tagging nucleic acid molecules in a biological specimen. The method involves using a solid substrate with multiple species of capture probes immobilized in distinct positions, wherein the capture probes have three components: a cleavage domain for releasing them from the substrate, a positional domain corresponding to their location on the substrate, and a capture domain. EP 3 916 108 A1 discloses to three main steps: (a) placing the solid substrate with capture probes in contact with the biological specimen, (b) allowing the capture probes to be released from the substrate, and (c) simultaneously or subsequently extending the released capture probes using nucleic acids from the biological specimen as templates.

EP 3 511 423 A1 The present invention relates to methods and products for localized or spatial detection and/or analysis of RNA in a tissue sample or a portion thereof, comprising: (a) providing an object substrate on which at least one species of capture probe, comprising a capture domain, is directly or indirectly immobilized such that the probes are oriented to have a free 3' end to enable said probe to function as a reverse transcriptase (RT) primer; (b) contacting said substrate with a tissue sample and allowing RNA of the tissue sample to hybridize to the capture probes; (c) generating cDNA molecules from the captured RNA molecules using said capture probes as RT primers; (d) labelling the cDNA molecules generated in step (c), wherein said labelling step may be contemporaneous with, or subsequent to, said generating step; (e) detecting a signal from the labelled cDNA molecules; and optionally (f) imaging the tissue sample, wherein the tissue sample is imaged before or after step (c).

US 6 485 944 B1 relates to methods of making and using immobilized arrays of nucleic acids, particularly methods for producing replicas of such arrays, including methods for producing high density arrays of nucleic acids and replicas of such arrays, as well as methods for preserving the resolution of arrays through rounds of replication.

US 8 865 404 B2 relates to a method for determining the nucleic acid sequence of a polynucleotide that is at least 1500 nucleotides in length comprising, inter alia, providing a polynucleotide; cleaving the polynucleotide into a plurality of oligonucleotide sequences; immobilizing only one end of each of the plurality of oligonucleotide; arranging the plurality of oligonucleotide sequences; attaching a label to a non-immobilized end of each of the plurality of oligonucleotide sequences; cleaving the plurality of oligonucleotide sequences to generate a plurality of labelled oligonucleotide fragments; collecting the plurality of oligonucleotide fragments; sequencing the plurality of oligonucleotide fragments to obtain oligonucleotide reads; assembling the oligonucleotide reads; and determining the nucleic acid sequence of the assembled oligonucleotide reads.

However, some current approaches require highly sensitive microscopes and microfluidics, hindering their application in clinical settings, and additionally, these approaches comprise a plurality of repetition-depend measurements which increases background and thus reduces signal-to-noise ratio (SNR).

### Summary

In light of the above, it is therefore an object of the present invention to overcome or at least to alleviated the shortcomings and disadvantages of the prior art. More particularly, it is an object of the present invention to provide a method and a corresponding system for measuring RNA at a cellular and subcellular level while retaining spatial context, which is simple enough to be feasibly applicable in non-specialist laboratories and in clinical settings for molecular diagnostics.

These objects are met by the present invention.

In a first aspect, the invention relates to a probe for RNA fluorescence in situ hybridization (FISH) comprising: a marker molecule, a targeting molecule comprising 22 to 55 nt comprising a spacer sequence, and a target binding sequence capable of binding to a target molecule, wherein the marker molecule may be connected to the targeting molecule via the spacer sequence.

It should be understood that the spacer sequence may also be referred to as spacer molecule, or simply as spacer.

Moreover, the probe may comprise a marker molecule binding sequence comprising 20 to 40 nt.

In one embodiment, the marker molecule may be bound to the marker molecule binding sequence, the marker molecule binding sequence may be connected to the spacer sequence, and the spacer sequence may be connected to the target binding sequence

In another embodiment, the probe may comprise an oligonucleotide molecule comprising the marker molecule binding sequence, the spacer sequence, and the target binding sequence.

Moreover, the spacer sequence may be less than 50% complementary to the target binding sequence.

In one embodiment, the marker molecule may comprise a DNA-nanostructure. Additionally or alternative, the DNA-nanostructure may comprise at least one of: a 2D DNA-nanostructure, and a 3D DNA-nanostructure.

In one embodiment, the marker molecule may comprise a protein. In another embodiment, the marker molecule may comprise an antibody. In a further embodiment, the marker molecule may comprise a nanoparticle. The nanoparticle may comprise gold particles. In an even further embodiment, the marker molecule may comprise a peptide. Moreover, the peptide may be a polypeptide such as a myc tag.

In one embodiment, the target molecule may comprise an RNA to be detected comprising an RNA target sequence.

Further, the probe may comprise a DNA oligonucleotide comprising a length between 57 and 92 nt. The DNA oligonucleotide may comprise the target binding sequence, wherein 20 to 55 nt of the DNA oligonucleotide may comprise the target binding sequence.

Moreover, the marker molecule may comprise at least one of: a fluorophore, a chromophore, a fluorescently labelled antibody, fluorescent silica nanobeads, fluorescent colloids, fluorescent melamine particles, a structure comprised of several oligonucleotides, a peptide, gold particles, and an aptamer.

In one embodiment, the targeting molecule may comprise a nucleotide sequence complementary to the RNA target sequence of the RNA to be detected.

In another embodiment, the marker molecule comprising the at least one fluorophore may comprise at least 10 fluorophore molecules, preferably at least 30 molecules, more preferably at least 40 fluorophore molecules incorporated in its structure. The marker molecule comprising the at least one fluorophore may comprise at most 80 fluorophore molecules, preferably at most 70 fluorophore molecules incorporated in its structure, such as 63 fluorophore molecules. For instance, when 3-4 intensity levels may be used, the probe may comprise anywhere between 10 and 63 fluorophore molecules in marker molecule. However, it should be understood that probes with lower intensity level may be possible, for example, probes with 10 fluorophores

Moreover, the marker molecule may comprise the at least one fluorophore comprising the DNA-nanostructure comprising the at least one fluorophore incorporated in its structure. The DNA-nanostructure may be at least one of: the 2D DNA-nanostructure, and the 3D DNA-nanostructure.

In one embodiment, the protein may comprise a fluorescent protein. The fluorescent protein may comprise a fluorescent antibody.

Moreover, the DNA-nanostructure may comprise a DNA design comprising a sequence of staples, wherein the sequence of staples may be configured to bind to a DNA scaffold to form the DNA-nanostructure. The sequence of staples configured to bind to the DNA scaffold forms the 2D DNA-nanostructure.

In another embodiment, the sequence of staples configured to bind to the DNA scaffold forms the 3D DNA-nanostructure.

When the marker molecule may comprise the DNA-nanostructure, the spacer sequence as part of the targeting molecule may be designed to directly and fixedly integrate into the DNA-nanostructure.

In general, 3D DNA-nanostructure constructs can indeed be considered thermostable, but this may be a relative term. The stability of the 3D DNA-nanostructure construct depends greatly on the conditions in which it's maintained. Typically, 3D DNA-nanostructure structures may be stable within a range of temperatures from 4°C to 60°C.

The buffer conditions can also greatly influence the thermostability of the structure. Most commonly, 3D DNA-nanostructure may be prepared in buffers containing monovalent (Na⁺, K⁺) and multivalent cations (Mg²⁺, Ca²⁺), which help stabilize the structure by screening the negative charges on the DNA backbone and facilitating the correct hybridization of the staples with the scaffold.

In terms of the "fixing" or "irreversibility" of staple binding to the scaffold, this may be more nuanced. DNA hybridization may be a dynamic process that can technically be reversed under certain conditions, such as high temperature or low salt concentrations, where the DNA strands can denature and the staples can dissociate from the scaffold. Therefore, it would not be strictly accurate to describe this process as "irreversible."

However, once the 3D DNA-nanostructure structure has been correctly folded, it may be relatively stable under appropriate conditions. The stability of the structure may be not just due to the individual binding of each staple strand to the scaffold, but also to the overall architecture of the structure, where every staple strand helps to stabilize its neighbors. In this sense, you could describe the process as being "kinetically trapped," where the properly folded structure may be a low-energy state that the system doesn't readily escape from.

Comparatively, the hybridization of two complementary 20-nucleotide single-stranded DNAs may be a simpler process and more susceptible to changes in conditions like temperature and salt concentration. This may be because it involves fewer base pairs and lacks the collective stability provided by the overall architecture in a 3D DNA-nanostructure structure. Thus, while the basic principles of DNA hybridization apply to both cases, the 3D DNA-nanostructure structure may be generally more stable than a single double-stranded DNA molecule.

Furthermore, at least one of: the target binding sequence, the spacer sequence, and the marker molecule binding sequence may be annealed to a complementary sequence of the DNA scaffold. A subsequence of at least one of: the target binding sequence, the spacer sequence, and the marker molecule binding sequence may be annealed to a complementary sequence of the DNA scaffold. The molecule annealed to the complementary sequence of the DNA scaffold may be molecule-DNA-scaffold annealed structure, wherein the molecule-DNA-scaffold annealed structure may be incorporated into the DNA-nanostructure. This may be particularly beneficial as the thermic and chemical stability of the annealing/binding may be elevated over the stability of a simple double-stranded DNA oligonucleotide.

In one embodiment, the set of probes may be linked to the marker molecule, wherein the marker molecule may be larger than a single fluorescence dye molecule moiety. This may be surprisingly advantageous, as due to being a large molecule, it can contain lots of fluorescence dye molecule moieties, which leads to a higher signal and a higher SNR compared to just one fluorophore

Further, the structure comprised of several oligonucleotides may comprise at least one branched DNA tree.

Moreover, the target binding sequence may comprise a complementarity to the subsequence of the RNA to be detected of at least 75%, preferably at least 85%, more preferably at least 95%. The targeting molecule may be complementary to a subsequence on one side of the DNA scaffold. The probe may comprise at least 2 targeting molecules, preferably at least 5 targeting molecules, wherein at least 2 of the targeting molecules may be complementary to a different sub-sequence of the DNA scaffold, preferably wherein at least 5 of the targeting molecules may be complementary to a different sub-sequence of the DNA scaffold. The probe may comprise 10 targeting molecules, wherein each of the 10 targeting molecules may be complementary to a different sub-sequence of the DNA scaffold.

When the marker molecule may comprise the DNA-nanostructure, the targeting molecule may be complementary to at least 20 nt and up to 40 nt of the 3D DNA-nanostructure structure.

When the marker molecule may comprise the DNA-nanostructure, the target binding sequence may be complementary to at least 20 nt and up to 40 nt of the DNA scaffold.

Moreover, incorporation of the targeting molecule into the structure of the DNA-nanostructure take place via Watson-Crick base pairing of a subsequence of the targeting molecule or the marker molecule binding sequence to the DNA scaffold or staple strands. This may be particularly beneficial, as it results in a binding that may be "kinetically trapped" and more stable compared to simple Watson-Crick base pairing of two double-stranded DNA oligonucleotides.

In one embodiment, the probe may comprise the marker molecule comprising the fluorescent DNA-nanostructure bound to a marker molecule binding sequence, wherein the marker molecule binding sequence may be connected to the spacer sequence, wherein the spacer sequence may be connected to the target binding sequence.

Furthermore, the probe may comprise the marker molecule comprising the fluorescent DNA-nanostructure bound to the oligonucleotide molecule.

In another embodiment, the 3D DNA-nanostructure may be a DNA origami.

Moreover, the probe may be configured to bind in situ to at least one sequence of the target molecule.

Additionally or alternatively, the probe may be for multiplexed detection of the target molecule. The multiplexing may be based on spectral barcoding of the target molecule. The multiplexing may be based on intensity barcoding of the target molecule.

In one embodiment, the probe may be for detecting microRNA.

In another embodiment, the marker molecule may be directly bound to the marker molecule binding sequence.

Moreover, the marker molecule binding sequence may be directly connected to the spacer molecule.

Further, the spacer molecule may be directly connected to the target binding sequence

In a further embodiment, the probe may comprise the marker molecule comprising the fluorescent DNA-nanostructure bound to the oligonucleotide molecule.

In one embodiment, at least one of: the marker molecule binding sequence, and the spacer sequence may be allocated between the marker molecule and the target binding sequence. The marker molecule binding sequence may be directly connected to the spacer sequence, and wherein the marker molecule binding sequence directly connected to the spacer sequence may be allocated between the marker molecule and the target binding sequence.

Furthermore, the targeting molecule may comprise at least one of: the marker molecule binding sequence, the spacer sequence and the target binding sequence. The targeting molecule may comprise the marker molecule binding sequence, the spacer sequence and the target binding sequence.

In a second aspect, the invention relates to a composition for RNA FISH comprising a set of probes for RNA FISH.

In one embodiment, the set of probes may comprise at least 1 probe for RNA FISH, preferably at least 5 probes for RNA FISH, more preferably at least 10 probes for RNA FISH.

In another embodiment, the set of probes may comprise at least one probe according to any of the preceding probe embodiments.

Moreover, the set of probes may comprise at least one probe binding to a sub-sequence of a target RNA sequence to result in at least one bound probe, wherein the at least one bound probe may be detectable using fluorescence microscopy. The set of probes may comprise at least two probes binding to the sub-sequence of the target RNA sequence to result in the at least two bound probes, wherein the at least two bound probes may be detectable using fluorescence microscopy.

In a further embodiment, the set of probes may comprise at least three probes binding to the sub-sequence of the target RNA sequence to result in the at least three bound probes, wherein the at least three bound probes may be detectable using fluorescence microscopy.

Furthermore, the set of probes may comprise at least four probes binding to the subsequence of the target RNA sequence to result in the at least four bound probes, wherein the at least four bound probes may be detectable using fluorescence microscopy.

Moreover, the sub-sequence of the target RNA sequence may be located within an open loop structure when folding the target RNA sequence under hybridization conditions.

In a third aspect, the invention relates to a method for designing a probe for RNA FISH for analyzing a sample.

In one embodiment, the method may comprise providing a probe for RNA FISH, wherein the probe may be according to any of the preceding probe embodiments.

In another embodiment, the method further may comprise detecting at least one target molecule in situ in the sample using the probe. The at least one target molecule may comprise an RNA to be detected.

The sample may comprise at least one of: cell, fixed cells, fixed mammalian tissues, fixed reptile tissues, ficed fish tissues, fresh-frozen tissue section, Formalin-Fixed Paraffin-Embedded (FFPE) section, and cryosection of a tissue.

Moreover, the method may comprise permantly linking the probe to a marker molecule.

In one embodiment, the method may comprise combining at least one flurophore molecule comprising a single color with one marker molecule linked to the probe with the target molecule, wherein the single color may comprise at least two intensity levels of occurrence, wherein each of the at least two intensity levels of occurrence direclty correlates to the number of fluorophore molecules per marker molecule.

In another embodiment, the method may comprise combining at least one fluorophore molecule comprising at least one color with one marker molecule linked to the probe with the target molecule, wherein the at least one color may comprise at least two different types of fluorophores. The method may comprise combining the at least one fluorophore molecule comprising two colors with one marker molecule linked to the probe with the target molecule, wherein each of the two colors may comprise a different type of fluorophores. This may be particularly advantageous, as it allows each of the two colors to occur only once.

Further, at least one of: each of the at least one color or each of the two colors may comprise at least two intensity levels of occurrence, wherein each of the at least two intensity levels of occurrence directly correlates to the number of fluorophore molecules per marker molecule. This may be notably advantegeous, as it leads, for instance, to 52 possible combinations for 4 different possible colors, in particular, for the two colors in one single probe, and it leads to 30 possible combinations, for instance, for 3 different colors in 4 different intensity levels, as well as for 4 different colors in 3 intensity levels.

Moreover, the method may comprise binding two probes to the target molecule, wherein the method may comprise labelling each of the two probes with the marker molecule comprising different colors, each color comprising a different type of fluorophore. Each of the two colors may comprise at least two intensity levels of occurrence, wherein each of the at least two intensity levels of occurrence directly correlates to the number of fluorophore molecules per marker molecule. This may be notably advantegeous, as it leads to 48 possible combinations for different possible colors, in particular, for the three colors.

In a further embodiment, the least one target molecule may be bound to the probe, wherein the method may comprise detecting the least one target molecule to probe. The method may comprise: imaging the sample, collecting at least one z-slice image, removing a background from the at least one z-slice image, identifying a number of individual, dot-like signals in a given channel, and counting the number of individual, dot-like signals identified in the given channel.

It should be understood that the individual, dot-like signal in a given channel may also be referred to as punctae.

The at least one z-slice image may comprise a plurality of images in a z distance fitting a resolution limit of a microscope.

Furthermore, removing the background may comprise removing the background from the at least one z-slice image by at least one background removal method. The at least one background removal method may comprise thresholding.

Moreover, the method may comprise recording in the given channel a signal from a single type of fluorophore comprising a distinct excitation and emission wavelength.

Additionally or alternatively, designing the probe may comprise comprising: defining via search in an mRNA repository a coding sequence (CDS) of a RNA of interest comprising a target RNA sequence, folding the target RNA sequence under hybridization conditions, and analyzing the RNA of interest for an open loop structure when folding the target RNA sequence under hybridization conditions. The mRNA repository may for example be a reference sequence database such as RefSeq. The method may comprise further at least one of: determining a probe length of n nucleotides, calculating a score for each n nucleotide sub-sequence, and estimating an average probability of a long sub-sequence to be an open loop. The calculating step may be performed iteratively in the CDS in single nucleotide increments for each stretch of n nucleotides.

Moreover, the method may comprise: selecting between 5 and 10 sub-sequences with the highest scores as probe targets, expanding the selected sub-sequence by 10 to 200 nt on each side to output stretched nucleotides, computationally probing the stretched nucleotides for matches of 22 nt to 50 nt probes, preferably at least 22 nt and less than 41 nt, more preferably less than 30 nt, and selecting matching sub-sequences based on thermodynamics comprising low likelihood of structure under buffer conditions of Na⁺ 300 mM and Mg²⁺ 11 mM.

Further, the method may comprise detecting microRNA.

In a fourth aspect, the invention relates to a process for producing a composition for RNA FISH, wherein the process may comprise producing at least one of: a probe recited herein, or a composition as recited herein. The process may comprise using any of method steps as recited herein.

In a fifth aspect, the invention relates to a kit for use in a molecular analysis, the comprising: a probe as recited herein, or a composition as recited herein. The probe may be in a solution. Further, the probe may be freeze-dried.

In one embodiment, the composition may comprise at least one probe as recited herein, wherein the at least one probe may be in a solution.

In a further embodiment, the composition may comprise at least one probe as recited herein, wherein the at least one probe may be freeze-dried.

Moreover, the kit may comprise at least one buffer for hybridization comprising at least one of: fixation buffer component, wash buffer, and hybridization buffer.

In a sixth aspect, the invention relates to a system configured to produce a probe for RNA FISH. The probe may be as recited herein.

In one embodiment, the system may be configured to produce a composition as recited herein.

In another embodiment, the system may be configured to produce a kit as recited herein.

In a further embodiment, the system may be configured to carry out any steps of the method as recited herein.

Moreover, the system may be configured to carry out any steps of the process as recited herein.

In a seventh aspect, the invention relates to use of the probe according as recited herein for quality control in gene therapy.

In one embodiment, the invention may comprise the use of the probe according as recited herein for quality control in siRNA/RNAi applications.

In another embodiment, the invention may comprise the use of the probe according as recited herein for determination of a cancer therapy for a specific tumor. The determination of the cancer therapy for the specific tumor may be based on at least one of: genetics, epigenetics, expression pattern, tumor-microenvironment, internal signal, and external signals.

Furthermore, the invention may comprise the use of the probe according as recited herein for determination of number and tissue/cell type/subcellular distribution of multiple RNAs simultaneously, without sequential hybridization steps.

In one embodiment, the invention may comprise the use of the probe according as recited herein in research to co-detect a set of at least one of: RNAs and proteins.

In a further embodiment, the invention may comprise the use of the probe according as recited herein for in research to co-detect a set of at least one of: mRNAs, and miRNAs.

Moreover, the invention may comprise the use of the composition as recited herein for quality control in gene therapy.

Further, the invention may comprise the use of the composition as recited herein for quality control in siRNA/RNAi applications.

Moreover, the invention may comprise the use of the composition as recited herein for determination of a cancer therapy for a specific tumor. The determination of the cancer therapy for the specific tumor may be all integrated in expression landscape of a tissue, wherein the determination may be based on at least one of: genetics, epigenetics, expression pattern, tumor-microenvironment, internal signal, and external signals.

Furthermore, the invention may comprise the use of the composition as recited herein for determination of number and tissue/cell type/subcellular distribution of multiple RNAs simultaneously, without sequential hybridization steps

Moreover, the invention may comprise the use of the composition as recited herein in research to co-detect a set of at least one of: RNAs and proteins.

Further, the invention may comprise the use of the composition as recited herein in research to co-detect a set of at least one of: mRNAs, and miRNAs.

In simple terms, some of the surprisingly advantageous features of the invention include: the possibility to link the target binding sequence to different types of marker molecules using the spacer molecule, so that different marker molecules can be deployed with the same target binding sequence; the length and composition of the target binding sequence, which enhances specificity and binding temperature (Tm, melting temperature) under hybridization conditions and thus allows for specific binding; the ability to bind only one to four probe as recited herein to a target DNA or RNA molecule, with this being sufficient for detection and visualization using fluorescent microscopy, and still achieving the required specificity and sensitivity, while conventional RNA FISH methods require 20-50 probes labelled with a single fluorescent moiety to bind to a target DNA or RNA molecule to make it detectable; use of a combination of marker molecules with different fluorophore species i.e., colors, per target RNA sequence. This combination can be achieved by combining several different fluorophore species within one marker molecule carrying multiple fluorophore molecules. Alternatively, this may be achieved by applying several different marker molecules binding to different subsequences of the same target RNA, in which case each marker molecule carries several molecules of one fluorophore species that differs from the fluorophore species in the other marker molecule(s) targeting the same RNA, in fluorescence emission. Alternatively, this may also be achieved by applying several different marker molecules which each carry fluorophore molecules of two or more different fluorophore species. Regardless of the way to achieve this, the goal is to achieve a color combination that is distinct and can be distinguished from other color combinations. This notably enables the detection of a much higher number of RNA species in the same experiment, without sequential hybridization rounds. This abbreviates and simplifies the experiment. Previously, this has only been possible through either using microfluidics, and/or super-resolution microscopy; due to the comparatively short binding sequence (20-55 nt, typically 30 nt) and the requirement of only at least 1 probe per target RNA for detection, partially degraded RNA can also be detected, which is particularly beneficial for tissue samples, especially FFPE tissue.

Moreover, the approach of the present invention is notably advantageous over the prior art, as it allows detecting and measuring up to 48 RNAs simultaneously, without sequential rounds of hybridization and washing. The approach of the method of the present invention comprises multiplexing based on spectral barcoding and intensity barcoding. Additionally, the unique molecular composition of the probes of the present invention comprises a plurality of fluorophore-containing structures incorporated in their structure such as fluorophore-conjugated oligonucleotides on the inside of the structure. This is notably and surprisingly advantageous, as it enables to perform a spectral barcoding and/or intensity barcoding in the context of RNA FISH. In particular, the approach of the present invention is surprisingly advantageous, as it lowers the requirements for analytical methods, for example, the state of the art could only achieve spectral bar coding by using super-resolution microscopy methods.

The approach of the present invention is also notably advantageous, as it merely requires identification of a type of sample, such as fixed cells, tissue, on which the probe of the present invention would be applied on; identification of the CDS and transcript version of a target RNA that is to be detected and the number of RNAs to be detected; and finding out an approximated abundance of the transcript expected in the target sample such as low, medium or high abundance or housekeeping gene.

Furthermore, the approach of the present invention is also surprisingly advantageous, as it allows to identify the ideal fluorophore color combination for each RNA to be detected such as weaker fluorophores that have low photon yield or appear in channels with typically high autofluorescence signal for high-abundance RNA targets. For each target RNA, the invention allows to choose a desired probe sequence, color(s) and/or color combination according to the expected abundance. Thus, the invention allows to output, in short time, a customized probe for RNA FISH. Such a customized probe can be delivered, for example, as a freeze-dried composition, which is readable readily used for analysis of samples, for instance by dissolving the freeze-dried probe is buffer solutions, as recited herein.

This invention is also notably advantageous, as it allows, for instance, DNA probes permanently linked to a marker molecule that is larger than a single fluorescence dye molecule moiety such as a fluorescent 2D or 3D DNA-nanostructure with over 10, even over 100 dye molecules incorporated into its structure, or a fluorescently labelled antibody, or other comparable structures.

Moreover, the invention allows to produce DNA probe structure comprising between 22 and 55 nucleotides target binding sequence with at least 95% complementarity to a subsequence within the target RNA sequence; a spacer molecule comprising between 4 and 40 nucleotides which are less than 50% complementary to a sub-sequence within the target RNA sequence, and a marker molecule binding sequence, which links the probe to the marker molecule. In the case of a DNA origami structure acting as marker molecule, the sequence is complementary to at least 20 and up to 40 nucleotides within the DNA origami structure, so that it is incorporated into that structure permanently via Watson-Crick-base pairing.

Moreover, the present invention permits that only 1-4 probes for RNA FISH may be required to bind to a sub-sequence of the target RNA sequence in order to give enough signal with a sufficient signal-to-noise ratio (SNR) for it to be detected using fluorescence microscopy. Binding of many more probes per target RNA is possible though, to massively increase signal. Furthermore, the present invention also allows locating the sub-sequence of the target RNA sequence on an open loop structure when folding the target RNA sequence under hybridization conditions.

Notably, the present invention also allows use fluorescent DNA origami as marker molecules for use within a FISH probe to detect mRNA molecules within fixed cells.

The present technology is also described by the following numbered embodiments.

Below, probe embodiments will be discussed. These embodiments are abbreviated by the letter "P" followed by a number. When reference is herein made to a probe embodiment, those embodiments are meant.
P1. A probe for RNA fluorescence in situ hybridization (FISH) comprising
   a marker molecule,
   a targeting molecule comprising 22 to 55 nt comprising a spacer sequence, and a
   target binding sequence capable of binding to a target molecule,
   wherein the marker molecule is connected to the targeting molecule via the spacer sequence.
P2. The probe according to the preceding embodiment, wherein the probe comprises a marker molecule binding sequence comprising 20 to 40 nt.
P3. The probe according to the two preceding embodiments, wherein
   the marker molecule is bound to the marker molecule binding sequence,
   the marker molecule binding sequence is connected to the spacer sequence, and
   the spacer sequence is connected to the target binding sequence
P4. The probe according to embodiments P1 and P2, wherein the probe comprises an oligonucleotide molecule comprising the marker molecule binding sequence, the spacer sequence, and the target binding sequence.
P5. The probe according to any of the two preceding embodiments, wherein the spacer sequence is less than 50% complementary to the target binding sequence.
P6. The probe according to any of the preceding embodiments, wherein the marker molecule comprises a DNA-nanostructure.
P7. The probe according to the preceding embodiment, wherein the DNA-nanostructure comprises at least one of:
   a 2D DNA-nanostructure, and
   a 3D DNA-nanostructure.
P8. The probe according to any of the preceding embodiments, wherein the marker molecule comprises a protein.
P9. The probe according to any of the preceding embodiments, wherein the marker molecule comprises an antibody.
P10. The probe according to any of the preceding embodiments, wherein the marker molecule comprises a nanoparticle.
P11. The probe according to the preceding embodiments, wherein the nanoparticle comprises gold particles.
P12. The probe according to any of the preceding embodiments, wherein the marker molecule comprises a peptide.
P13. The probe according to the preceding embodiment, wherein the peptide is a polypeptide such as a myc tag.
P14. The probe according to any of the preceding embodiments, wherein the target molecule comprises an RNA to be detected comprising an RNA target sequence.
P15. The probe according to any of the preceding embodiments, wherein the probe comprises a DNA oligonucleotide comprising a length between 57 and 92 nt.
P16. The probe according to the preceding embodiment, wherein the DNA oligonucleotide comprises the target binding sequence, wherein 20 to 55 nt of the DNA oligonucleotide comprises the target binding sequence.
P17. The probe according to any of the preceding embodiments, wherein the marker molecule comprises at least one of: a fluorophore, a chromophore, a fluorescently labelled antibody, fluorescent silica nanobeads, fluorescent colloids, fluorescent melamine particles, a structure comprised of several oligonucleotides, a peptide, gold particles, and an aptamer.
P18. The probe according to any of the preceding embodiments and with the features of embodiment P14, wherein the targeting molecule comprises a nucleotide sequence complementary to the RNA target sequence of the RNA to be detected.
P19. The probe according to any of the preceding embodiments and with the features of embodiment P17, wherein the marker molecule comprising the at least one fluorophore comprises at least 10 fluorophore molecules, preferably at least 30 molecules, more preferably at least 40 fluorophore molecules incorporated in its structure.
P20. The probe according to the preceding embodiment, wherein the marker molecule comprising the at least one fluorophore comprises at most 80 fluorophore molecules, preferably at most 70 fluorophore molecules incorporated in its structure, such as 63 fluorophore molecules.
P21. The probe according to any of the two preceding embodiments with the feature of embodiment P6, wherein the marker molecule comprises the at least one fluorophore comprising the DNA-nanostructure comprising the at least one fluorophore incorporated in its structure.
P22. The probe according to the preceding embodiment and with the features of embodiment P7, wherein the DNA-nanostructure is at least one of:
   the 2D DNA-nanostructure, and
   the 3D DNA-nanostructure.
P23. The probe according to any of the preceding embodiments and with the features of embodiment P8, wherein the protein comprises a fluorescent protein.
P24. The probe according to the preceding embodiment, wherein the fluorescent protein comprises a fluorescent antibody.
P25. The probe according to any of the preceding embodiments and with the features of embodiment P6, wherein the DNA-nanostructure comprises a DNA design comprising a sequence of staples, wherein the sequence of staples is configured to bind to a DNA scaffold to form the DNA-nanostructure.
P26. The probe according to the preceding embodiments and with the features of embodiment P7, wherein the sequence of staples configured to bind to the DNA scaffold forms the 2D DNA-nanostructure.
P27. The probe according to any of the two preceding embodiments and with the features of embodiment P7, wherein the sequence of staples configured to bind to the DNA scaffold forms the 3D DNA-nanostructure.
P28. The probe according to any of the preceding embodiments and with the features of embodiments P6 or P7, wherein when the marker molecule comprises the DNA-nanostructure, the spacer sequence as part of the targeting molecule is designed to directly and fixedly integrate into the DNA-nanostructure.
P29. The probe according to any of the preceding embodiments and with the features of embodiment P25, wherein at least one of: the target binding sequence, the spacer sequence, and the marker molecule binding sequence is annealed to a complementary sequence of the DNA scaffold.
P30. The probe according to the preceding embodiment, wherein a subsequence of at least one of: the target binding sequence, the spacer sequence, and the marker molecule binding sequence is annealed to a complementary sequence of the DNA scaffold.
P31. The probe according to the preceding embodiment, wherein the molecule annealed to the complementary sequence of the DNA scaffold is molecule-DNA-scaffold annealed structure, wherein the molecule-DNA-scaffold annealed structure is incorporated into the DNA-na nostructure.
P32. The probe according to any of the preceding embodiments, wherein the probe is linked to the marker molecule, wherein the marker molecule is larger than a single fluorescence dye molecule moiety.
P33. The probe according to any of the preceding embodiments and with the features of embodiment P17, wherein the structure comprised of several oligonucleotides comprises at least one branched DNA tree.
P34. The probe according to any of the preceding embodiments and with the feature of embodiment P18, wherein the target binding sequence comprises a complementarity to the subsequence of the RNA to be detected of at least 75%, preferably at least 85%, more preferably at least 95%.
P35. The probe according to the preceding embodiment and with the feature of embodiment P22, wherein the spacer molecule is complementary to a subsequence on one side of the DNA scaffold.
P36. The probe according to the preceding embodiment, wherein the probe comprises at least 2 spacer molecules, preferably at least 5 spacer molecules, wherein at least 2 of the spacer molecules is complementary to a different sub-sequence of the DNA scaffold, preferably wherein at least 5 of the spacer molecules is complementary to a different subsequence of the DNA scaffold.
P37. The probe according to the preceding embodiment, wherein the probe comprises 10 spacer molecules, wherein each of the 10 spacer molecules is complementary to a different sub-sequence of the DNA scaffold.
P38. The probe according to any of the preceding embodiments and with features of embodiment P28, wherein when the marker molecule comprises the DNA-nanostructure, the targeting molecule is complementary to at least 20 nt and up to 40 nt of the 3D DNA-nanostructure structure.
P39. The probe according to any of the preceding embodiments and with features of embodiment P28, wherein when the marker molecule comprises the DNA-nanostructure, the target binding sequence is complementary to at least 20 nt and up to 40 nt of the DNA scaffold.
P40. The probe according to any of the two preceding embodiments or according to embodiment P28, wherein incorporation of the targeting molecule into the structure of the DNA-nanostructure take place via Watson-Crick base pairing of a subsequence of the targeting molecule or the marker molecule binding sequence to the DNA scaffold or staple strands.
P41. The probe according to any of the preceding embodiments, wherein the probe comprises the marker molecule comprising the fluorescent DNA-nanostructure bound to a marker molecule binding sequence, wherein the marker molecule binding sequence is connected to the spacer sequence, wherein the spacer sequence is connected to the target binding sequence.
P42. The probe according to any of the preceding embodiments and with the features of embodiment P4, wherein the probe comprises the marker molecule comprising the fluorescent DNA-nanostructure bound to the oligonucleotide molecule.
P43. The probe according to any of the preceding embodiments and with the features of embodiment P7, wherein the 3D DNA-nanostructure is a DNA origami.
P44. The probe according to any of the preceding embodiments, wherein the probe is configured to bind in situ to at least one sequence of the target molecule.
P45. The probe according to any of the preceding embodiments, wherein the probe is for multiplexed detection of the target molecule.
P46. The probe according to the preceding embodiment, wherein the multiplexing is based on spectral barcoding of the target molecule.
P47. The probe according to the preceding embodiment, wherein the multiplexing is based on intensity barcoding of the target molecule.
P48. The probe according to any of the preceding embodiments, wherein the probe is for detecting microRNA.
P49. The probe according to any of the preceding embodiments and with the features of embodiment P3, wherein the marker molecule is directly bound to the marker molecule binding sequence.
P50. The probe according to any of the preceding embodiments and with the features of embodiment P3, wherein the marker molecule binding sequence is directly connected to the spacer molecule.
P51. The probe according to any of the preceding embodiments and with the features of embodiment P3, wherein the spacer molecule is directly connected to the target binding sequence
P52. The probe according to any of the preceding embodiments and with the features of embodiment P4, wherein the probe comprises the marker molecule comprising the fluorescent DNA-nanostructure bound to the oligonucleotide molecule.
P53. The probe according to any of the preceding embodiments and with the features of embodiment P3, wherein at least one of: the marker molecule binding sequence, and the spacer sequence is allocated between the marker molecule and the target binding sequence.
P54. The probe according to the preceding embodiment, wherein the marker molecule binding sequence is directly connected to the spacer sequence, and wherein the marker molecule binding sequence directly connected to the spacer sequence is allocated between the marker molecule and the target binding sequence.
P55. The probe according to any of the preceding embodiments, wherein the targeting molecule comprises at least one of: the marker molecule binding sequence, the spacer sequence and the target binding sequence.
P56. The probe according to the preceding embodiment, wherein the targeting molecule comprises the marker molecule binding sequence, the spacer sequence and the target binding sequence.

Below, composition embodiments will be discussed. These embodiments are abbreviated by the letter "C" followed by a number. When reference is herein made to a composition embodiment, those embodiments are meant.
C1. A composition for RNA FISH comprising a set of probes for RNA FISH.
C2. The composition according to the preceding embodiment, wherein the set of probes comprises at least 1 probe for RNA FISH, preferably at least 5 probes for RNA FISH, more preferably at least 10 probes for RNA FISH.
C3. The composition according to any of the two preceding embodiments, wherein the set of probes comprises at least one probe according to any of the preceding probe embodiments.
C4. The composition according to any of the preceding composition embodiments, wherein the set of probes comprises at least one probe binding to a sub-sequence of a target RNA sequence to result in at least one bound probe, wherein the at least one bound probe is detectable using fluorescence microscopy.
C5. The composition according to the preceding composition embodiment, wherein the set of probes comprises at least two probes binding to the sub-sequence of the target RNA sequence to result in the at least two bound probes, wherein the at least two bound probes are detectable using fluorescence microscopy.
C6. The composition according to any of the two the preceding composition embodiments, wherein the set of probes comprises at least three probes binding to the sub-sequence of the target RNA sequence to result in the at least three bound probes, wherein the at least three bound probes are detectable using fluorescence microscopy.
C7. The composition according to any of the three preceding composition embodiments, wherein the set of probes comprises at least four probes binding to the sub-sequence of the target RNA sequence to result in the at least four bound probes, wherein the at least four bound probes are detectable using fluorescence microscopy.
C8. The composition according to any of the four preceding embodiments, wherein the sub-sequence of the target RNA sequence is located within an open loop structure when folding the target RNA sequence under hybridization conditions.

Below, method embodiments will be discussed. These embodiments are abbreviated by the letter "M" followed by a number. When reference is herein made to a method embodiment, those embodiments are meant.
M1. A method for designing a probe for RNA FISH for analyzing a sample.
M2. The method according to the preceding embodiment, wherein the method comprises providing a probe for RNA FISH, wherein the probe is according to any of the preceding probe embodiments.
M3. The method according to any of the preceding method embodiments, wherein the method further comprises detecting at least one target molecule in situ in the sample using the probe.
M4. The method according to the preceding embodiment, wherein the at least one target molecule comprises an RNA to be detected.
M5. The method according to any of the preceding embodiments, wherein the sample comprises at least one of: cell, fixed cells, fixed mammalian tissues, fixed reptile tissues, ficed fish tissues, fresh-frozen tissue section, Formalin-Fixed Paraffin-Embedded (FFPE) section, and cryosection of a tissue.
M6. The method according to any of the preceding method embodiments, wherein the method comprises permantly linking the probe to a marker molecule.
M7. The method according to any of the preceding method embodiments, wherein the method comprises combining at least one flurophore molecule comprising a single color with one marker molecule linked to the probe with the target molecule, wherein the single color comprises at least two intensity levels of occurrence, wherein each of the at least two intensity levels of occurrence direclty correlates to the number of fluorophore molecules per marker molecule.
M8. The method according to any of the preceding method embodiments, wherein the method comprises combining at least one fluorophore molecule comprising at least one color with one marker molecule linked to the probe with the target molecule, wherein the at least one color comprises at least two different types of fluorophores.
M9. The method according to the preceding method embodiments, wherein the method comprises combining the at least one fluorophore molecule comprising two colors with one marker molecule linked to the probe with the target molecule, wherein each of the two colors comprises a different type of fluorophores.
M10. The method according to any of the two preceding embodiments, wherein at least one of:
   each of the at least one color or each of the two colors comprises at least two intensity levels of occurrence, wherein each of the at least two intensity levels of occurrence directly correlates to the number of fluorophore molecules per marker molecule.
M11. The method according to any of the preceding method embodiments, wherein the method comprises binding two probes to the target molecule, wherein the method comprises labelling each of the two probes with the marker molecule comprising different colors, each color comprising a different type of fluorophore.
M12. The method according to the preceding embodiment, wherein each of the two colors comprises at least two intensity levels of occurrence, wherein each of the at least two intensity levels of occurrence directly correlates to the number of fluorophore molecules per marker molecule.
M13. The method according to any of the preceding method embodiments and with the features of embodiment M3, wherien the least one target molecule is bound to the probe, wherein the method comprises detecting the least one target molecule to probe.
M14. The method according to the preceding embodiment, wherein the method comprises imaging the sample,
   collecting at least one z-slice image,
   removing a background from the at least one z-slice image,
   identifying a number of individual, dot-like signals in a given channel, and
   counting the number of individual, dot-like signals identified in the given channel.
M15. The method according to the preceding embodiment, wherein the at least one z-slice image comprises a plurality of images in a z distance fitting a resolution limit of a microscope.
M16. The method according to any of the two preceding embodiments, wherein removing the background comprises removing the background from the at least one z-slice image by at least one background removal method.
M17. The method according to the preceding embodiment, wherein the at least one background removal method comprises thresholding.
M18. The method according to any of three embodiments, wherein the method comprises recording in the given channel a signal from a single type of fluorophore comprising a distinct excitation and emission wavelength.
M19. The method according to any of the preceding method embodiments, wherein designing the probe comprises
   defining via search in an mRNA repository a coding sequence (CDS) of a RNA of interest comprising a target RNA sequence,
   folding the target RNA sequence under hybridization conditions, and
   analyzing the RNA of interest for an open loop structure when folding the target RNA sequence under hybridization conditions.
M20. The method according to the preceding embodiment, wherein the method comprises further at least one of:
   determining a probe length of n nucleotides,
   calculating a score for each n nucleotide sub-sequence, and
   estimating an average probability of a long sub-sequence to be an open loop.
M21. The method according to the preceding embodiment, wherein the calculating step is performed iteratively in the CDS in single nucleotide increments for each stretch of n nucleotides.
M22. The method according to any of the two preceding embodiments, wherein the method comprises
   selecting between 5 and 10 sub-sequences with the highest scores as probe targets,
   expanding the selected sub-sequence by 10 to 200 nt on each side to output stretched nucleotides,
   computationally probing the stretched nucleotides for matches of 22 nt to 50 nt probes, preferably at least 22 nt and less than 41 nt, more preferably less than 30 nt, and
   selecting matching sub-sequences based on thermodynamics comprising low likelihood of structure under buffer conditions of Na⁺ 300 mM and Mg²⁺ 11 mM.
M23. The method according to any of the preceding method embodiments, wherein the method comprises detecting microRNA.

Below, production process embodiments will be discussed. These embodiments are abbreviated by the letter "PP" followed by a number. When reference is herein made to a production process embodiment, those embodiments are meant.
PP1. A process for producing a composition for RNA FISH, wherein the process comprises producing at least one of
   a probe according to any of the preceding probe embodiments, or
   a composition according to any of the preceding composition embodiments.
PP2. The process according to the preceding embodiment, wherein the process comprises using any of method steps according to any of the preceding method embodiments.

Below, kit embodiments will be discussed. These embodiments are abbreviated by the letter "K" followed by a number. When reference is herein made to a kit embodiment, those embodiments are meant.
K1. A kit for use in a molecular analysis, the comprising
   a probe according to any of the preceding probe embodiments, or
   a composition according to any of the preceding composition embodiments.
K2. The kit according to the preceding embodiment, wherein the probe is in a solution.
K3. The kit according to any of the two preceding embodiments, wherein the probe is freeze-dried.
K4. The kit according to any of the preceding kit embodiments, wherein the composition comprises at least one probe according to any of the preceding probe embodiments, wherein the at least one probe is in a solution.
K5. The kit according to any of the preceding kit embodiments, wherein the composition comprises at least one probe according to any of the preceding probe embodiments, wherein the at least one probe is freeze-dried.
K6. The kit according to any of the preceding kit embodiments, wherein the kit comprises at least one buffer for hybridization comprising at least one of
   fixation buffer component,
   wash buffer, and
   hybridization buffer.

Below, system embodiments will be discussed. These embodiments are abbreviated by the letter "S" followed by a number. When reference is herein made to a system embodiment, those embodiments are meant.
S1. A system configured to produce a probe for RNA FISH.
S2. The system according to the preceding embodiment, wherein the probe is according to any of the preceding probe embodiments.
S3. The system according to any of the preceding system embodiments, wherein the system is configured to produce a composition according to any of the preceding composition embodiments.
S4. The system according to any of the preceding system embodiments, wherein the system is configured to produce a kit according to any of the preceding kit embodiments.
S5. The system according to any of the preceding system embodiments, wherein the system is configured to carry out any steps of the method according to any of the preceding method embodiments.
S6. The system according to any of the preceding system embodiments, wherein the system is configured to carry out any steps of the process according to any of the preceding process embodiments.

Below, use embodiments will be discussed. These embodiments are abbreviated by the letter "U" followed by a number. When reference is herein made to a use embodiment, those embodiments are meant.
U1. Use of the probe according to any of the preceding probe embodiments for quality control in gene therapy.
U2. Use of the probe according to any of the preceding probe embodiments for quality control in siRNA/RNAi applications.
U3. Use of the probe according to any of the preceding probe embodiments for determination of a cancer therapy for a specific tumor.
U4. Use of the probe according to the preceding embodiment, wherein the determination of the cancer therapy for the specific tumor is based on at least one of: genetics, epigenetics, expression pattern, tumor-microenvironment, internal signal, and external signals.
U5. Use of the probe according to any of the preceding probe embodiments for determination of number and tissue/cell type/subcellular distribution of multiple RNAs simultaneously, without sequential hybridization steps.
U6. Use of the probe according to any of the preceding probe embodiments in research to co-detect a set of at least one of: RNAs and proteins.
U7. Use of the probe according to any of the preceding probe embodiments in research to co-detect a set of at least one of: mRNAs, and miRNAs.
U8. Use of the composition according to any of the preceding composition embodiments for quality control in gene therapy.
U9. Use of the composition according to any of the preceding composition embodiments for quality control in siRNA/RNAi applications.
U10. Use of the composition according to any of the preceding composition embodiments for determination of a cancer therapy for a specific tumor.
U11. Use of the composition according to the preceding embodiment, wherein the determination of the cancer therapy for the specific tumor is all integrated in expression landscape of a tissue, wherein the determination is based on at least one of: genetics, epigenetics, expression pattern, tumor-microenvironment, internal signal, and external signals.
U12. Use of the composition according to any of the preceding composition embodiments for determination of number and tissue/cell type/subcellular distribution of multiple RNAs simultaneously, without sequential hybridization steps
U13. Use of the composition according to any of the preceding composition embodiments in research to co-detect a set of at least one of: RNAs and proteins.
U14. Use of the composition according to any of the preceding composition embodiments in research to co-detect a set of at least one of: mRNAs, and miRNAs.

The present invention will now be described with reference to the accompanying drawings which illustrate embodiments of the invention. These embodiments should only exemplify, but not limit, the present invention.
- Fig. 1: conceptually depicts a typical RNA FISH protocol compared to the probe according to embodiment of the present invention;
- Fig. 2: schematically depicts a probe for RNA FISH according to embodiments of the present invention;
- Fig. 3: schematically depicts a probe for RNA comprising a DNA-nanostructure according to embodiments of the present invention;
- Fig. 4: schematically depicts a detailed view of a probe for RNA comprising a DNA-nanostructure according to embodiments of the present invention;
- Fig. 5: depicts a multiplex detection of three cancer biomarkers;
- Fig. 6: depicts a multiplex detection of 5 cancer biomarker RNAs with one probe according to embodiments of the present invention;
- Fig. 7: depicts images of an example of false-color labelling of multi-color probe according to embodiments of the present invention.

It is noted that not all the drawings carry all the reference signs. Instead, in some of the drawings, some of the reference signs have been omitted for sake of brevity and simplicity of illustration. Embodiments of the present invention will now be described with reference to the accompanying drawings.

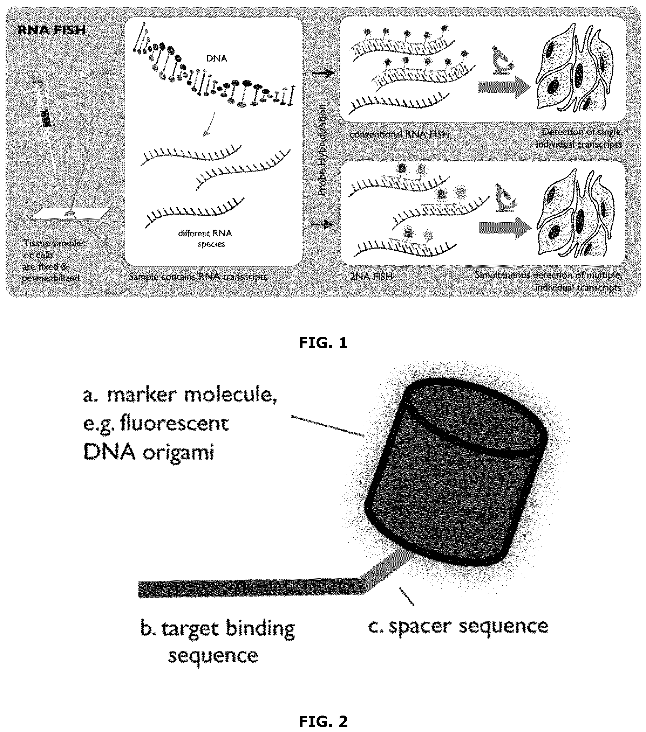

Fig. 1 conceptually depicts a typical RNA FISH protocol compared to the probe according to embodiment of the present invention. The probe according to embodiments of the present invention enables measuring several RNAs (up to 52) simultaneously in fixed cell or tissue samples with spatial context. First, the tissue sample or cells are fixed and permeabilized. These contain both DNA and RNA, of which in RNA FISH only the RNA will be detected. RNA FISH probes with a binding sequence partially complementary to the target RNA to be measured are added to the sample, followed by hybridization over 10-16 hours at a specific temperature (e.g., 30°C) in hybridization buffer. The rightmost panels show the difference between conventional RNA FISH and the probe of the present invention. Upper panel: Conventional RNA FISH probes using 20-50 short DNA oligonucleotides modified with one fluorophore molecule per oligonucleotide. These allow detection of only one RNA per fluorescence channel. In a typical epifluorescence microscope, this limits the number of RNAs to be detected simultaneously to 3-4. Lower Panel: probe according to embodiments of the present invention can use e.g., two DNA oligonucleotide probes using e.g., a fluorescent 3D DNA Origami as a fluorescent marker molecule. By combining different fluorescent markers with divergent excitation and emission spectra, and a specific number of fluorophore molecules within the fluorescent marker molecule, up to 52 RNAs can be measured simultaneously in one hybridization.

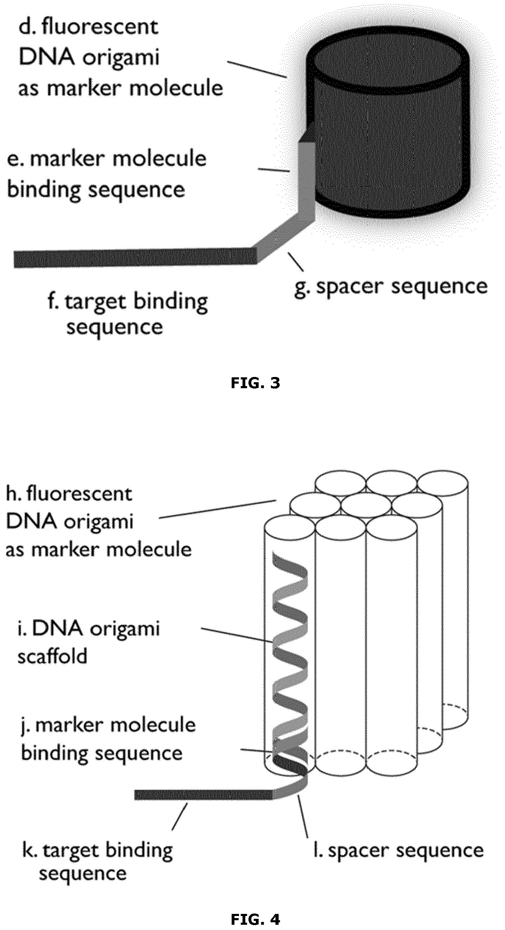

Figs. 2-4 schematically depicts the makeup of a probe according to embodiments of the present invention. In simple terms, the probe comprises a (a) marker molecule (e.g., a fluorescent DNA origami), connected via a (c) spacer sequence on the targeting molecule to the (b) target binding sequence. The spacer sequence and target binding sequence are comprised by the targeting molecule. Fig. 3 conceptually depicts a probe using a (d) fluorescent DNA origami as a marker molecule, which is bound to an (e) marker molecule binding sequence, connected to a (g) spacer sequence, connected to (f) the target binding sequence. The targeting molecule comprises (e)-(g). Fig. 4 depicts a detailed view of Fig. 3. Inside the DNA origami molecule (h) is shown the single-stranded DNA origami scaffold (i), which is bound via Watson-Crick pairing and integration into the 3D structure to the targeting molecule by the (j) marker molecule binding sequence, linked to the spacer sequence (I) and the target binding sequence (k). The targeting molecule comprises (j)-(I).

Fig. 2 schematically depicts a probe for RNA FISH according to embodiments of the present invention. The probe for RNA FISH may also referred to simply a probe. In simple terms, the probe comprises: a marker molecule, and a targeting molecule. The targeting molecule comprises at least a spacer sequence and a target binding sequence.

In one embodiment, the marker molecule may be directly connected to the spacer sequence on a first end of the targeting molecule. Moreover, the targeting molecule comprises a spacer sequence connected to a target binding sequence on a second end of the targeting molecule. Put differently, the probe may comprise the targeting molecule with the spacer sequence arranged between the marker molecule and the target binding sequence.

In one embodiment, the marker may comprise a fluorescent structure such as a fluorescent DNA-nanostructure. The DNA-nanostructure may be a 2D and/or 3D DNA-nanostructure. In one embodiment, the marker molecule may also comprise a DNA origami.

In more simple words, Fig. 2 depicts a makeup of a probe for RNA FISH comprising: a marker molecule such a fluorescent 3D DNA-nanostructure, connected via a spacer sequence to a target binding sequence.

Fig. 3 schematically depicts a probe for RNA comprising a DNA-nanostructure according to embodiments of the present invention. In simple terms, Fig. 3 depicts the probe of Fig. 2 comprising a fluorescent 3D DNA-nanostructure as a marker molecule, which is bound to a marker molecule binding sequence connected to a spacer molecule which is connected to the target binding sequence.

It should be understood that Fig. 3 also intends to conceptually depict one oligonucleotide which comprises the marker molecule binding sequence, the spacer sequence, and the target binding sequence. This nucleotide may also be referred to as the targeting molecule. Furthermore, it should be understood that when such one oligonucleotide is referred to, only the last part of it, i.e., the target binding sequence, actually binds to the target RNA and therefore has to be complementary to it.

Fig. 4 schematically depicts a detailed view of Fig. 3. In simple terms, inside the 3D DNA-nanostructure molecule is shown a single-stranded 3D DNA-nanostructure scaffold which is bound via Watson-Crick pairing and integrated into the 3D structure by the marker molecule binding sequence linked to the spacer molecule and the target binding sequence.

### Example 1: General example to produce the probe according to embodiments of the present invention

In one embodiment, the probe may comprise a DNA-nanostructure as a marker molecule, for instance, a DNA origami. To achieve such a probe, it is necessary to perform the following steps:
Firstly, the probe should be designed to determine which target binding sequences are best for a desired target RNA. To do this, a coding sequence of a target RNA sequence may be input into a software, which then may out put a ranked list of potential binding sequences. The most suitable binding sequence may be selected, which may be acquired as single-stranded DNA oligonucleotides.

Second, the selected binding sequence may be dissolved in RNAse-free H₂O. Such a sequence may also be referred to as handles. Then, the dissolved handles are mixed with a pre-made master mix for the desired, e.g., DNA origami as marker molecule. This master mix may include the scaffold, as well as an abundance of short ssDNA oligonucleotides which also be referred to as staples, which be divided into colorless staples without dye molecules ("core mix"), and staples with a fluorescence dye molecule attached at the 3' end of the ssDNA oligonucleotide ("dye mix"). This mix may also include a folding buffer with 22 mM MgCl₂ (FoB22), which consists of 5 mM Tris, 1 mM EDTA, 22 mM MgCl₂, 5 mM NaCl, and RNAse-free water. It should be understood that these concentrations are final concentrations in the mix. The buffer may, usually, be made as a 10x stock. All of the reagents mentioned must be RNAse-free. Each mix may, e.g., be created at 50 µl scale, but it should be understood that this may be scaled up.

Third, then the DNA origamis-handle-buffer mix is subjected to an annealing temperature gradient in a PCR machine.

Fourth, the now folded probes, in this example DNA origamis + handles, may be purified, for instance, by either using a 2% agarose gel electrophoresis, or preferably, using Amicon 100 kDa filtration columns.

**Table 1.1 Example of Buffer B**

| **Component** | **Stock cone.** | **Volume / amount** | **Final cone.** |
|---|---|---|---|
| Tris (pH8) RNAse free 1M | 1M | 250 µl | 5 mM |
| EDTA RNAse free 0.5M | 0.5M | 100 µl | 1 mM |
| MgCl₂ RNAse free 1M | 1M | 500 µl | 10 mM |
| H₂O RNAse free | | fill to 50 ml | |
| Total: | | 50 ml | |

The resulting probe may be then quality-controlled by running an aliquot of the prepurification and post-purification probe on a 2% analytical agarose gel and verifying band size and clarity.

The resulting liquid can then be provided to a user, ideally frozen or at 4°C or freeze-dried.

### Example 2: exemplary steps on an experiment on a sample type comprising fixed cells from cell culture.

The following is an example of the use of the probe according to embodiments of the present invention on a sample comprising fixed cells from a cell culture.

For the fixation and permeabilization, the cells required are seeded at a low to medium density in a suitable vessel, for example, Ibidi µ-Slide or a glass-bottom 96-well plate suitable for high resolution microscopy. The cells are then fixed after growing for a certain number of days, e.g., via methanol or formaldehyde fixation. For instance, pre-chill 70% (v/v) EtOH in RNAse-free H₂O at -20°C or -80°C, once cells have grown to the desired density and at least 24h have passed from seeding, aspirate the growth medium, wash once with 1X PBS, add 150 µL of fixation solution to each well containing cells and incubate at room temperature for 10 min. Do not over fixate the cells; rather make it 8 min than exceeding 10min, wash twice with 1x PBS, add ice-cold 70% (v/v) EtOH in RNAse-free water and store at 4°C (at least overnight, up to one week), and after permeabilization in 70% EtOH, move to the hybridization steps.

The hybridization may comprise a plurality of steps, for instance, washing with a buffer, storage after the washing, a hybridization with a buffer, a second washing and imaging. In simple words, the hybridization comprises preparing a wash buffer (freshly prepare for a day), preparing a hybridization solution which may, for instance, comprise a dilute probe for RNA as desired with a buffer B, then mixing with 2x hybridization buffer 1+1. Removing the 70% ethanol from the sample, adding 150 µl of the wash buffer (e.g., same formamide (FA) % as subsequent Hybridization Buffer) and let standing for approximately 5min at, for example, 30°C. Then, wash buffer is removed and 150 µl hybridization solution to each well is added, and covered with Seal Ibidi slide with parafilm and tin foil to protect from light. Subsequently, Incubate at hybridization temperature, e.g., 30°C, possibly 30-65°C, overnight in the dark such as in an incubator or slide incubator.

Afterwards, the hybridization solution is removed and 150 µl of wash buffer (same FA % as Hyb solution) is added and swirled for a short time and removed. This comprises further wrapping tin foil around the hybridized sample, incubating it for, for example, 30 mint at 30°C in the dark. Afterwards, it takes place removing the wash buffer, adding a Hoechst solution such 0.5 µL Hoechst in 5mL of wash buffer, 1:10.000), wrapping in tin foil and incubating at 30°C for 30min. Then, the removing and adding of 150 µL sterile-filtered Sodium Citrate-Sodium Chloride Solution (SSC). It should be understood that 1x intends to refer to the 1x concentrated buffer (final use concentration). For instance, SSC buffer is usually prepared at a higher concentration, such as 20x, which means that it has to be diluted 1:20 in order to reach a 1x concentration.

Finally, it is performed the imaging on an epifluorescence microscope with z-stacks of ~200nm or less (at or below diffraction limit), wherein using appropriate filters according to dyes used in the probes, and subsequently, an analysis of the images is performed.

The tables below display data relating to the hybridization example explained above.

**Table 2.1 Components for hybridization**

| **Component** | **Stock conc.** | **Supplier** | **Cat. No.** | **Volume / amount** | **Final conc.** | **Where** |
|---|---|---|---|---|---|---|
| Formaldehyde | 37% (v/v) | | | 100 µl | 10% | |
| PBS RNAse-free | 10x | | | 100 µl | 1x | |

| **Component** | **Stock cone.** | **Supplier** | **Cat. No.** | **Volume / amount** | **Final conc.** | **Where** |
|---|---|---|---|---|---|---|
| RNASe-free water | - | | 800 | 800 µl | - | |
| Total: | | | | 1 ml | | |

**Table 2.2 Wash buffer**

| **Component** | **Stock cone.** | **Supplier** | **Cat. No.** | **Volume / amount** | **Final conc.** | |
|---|---|---|---|---|---|---|
| SSC | 20x | Ambion/Invitrogen | AM9770 | 500 µl | 2x | |
| Formamide, deionized¹ | 100% | Carl Roth | P04.1 | 500 ~ µl | 1 0% | 4°C |
| RNASe-free water | | | | 4 ml | | |
| Total: | | | | 5 ml | | |

**Table 2.3 Wash buffer: Storage**

| **Component** | **Stock cone.** | **Supplier** | **Cat. No.** | **Volume / amount** | **Final conc.** | |
|---|---|---|---|---|---|---|
| SSC | 20x | Ambion/Invitrogen | AM9770 | 500 µl | 2x | |
| Formamide, deionized¹ | 100% | Carl Roth | P04.1 | 500 ~ µl | 10% | 4°C |
| RNASe-free water | | | | 4 ml | | |
| Total: | | | | 5 ml | | |

**Table 2.4 Hybridization buffer**

| **Component** | **Stock cone.** | **Supplier** | **Cat. No.** | **Volume / amount** | **Final cone.** | |
|---|---|---|---|---|---|---|
| Dextran sulfate² | | Sigma/Merck | D8906 | 400 mg | 20% | |
| E. coli RNA | | | | 4 mg | 0,5 mg/ml | |
| Vanadyl Ribonucleoside complex | 200 mM | NEB | S1402S | 40 µl | 2 mM | |
| BSA (UltraPure) | 50 mg/ml | Ambion/Invitrogen | AM2616 | 1.6 µl | 0.02 mg/ml | |
| SSC | 20x | Ambion/Invitrogen | AM9770 | 800 µl | 4X | |
| Formamide, deionized¹ | 100% | Carl Roth | P04.1 | 400 µl | 10% | |
| ***Only for probe according to embodiments of the present inventions:** MgCl_{2 (leave out for normal Stellaris RNA FISH)}* | 1M | | | *44 µl* | *11 mM* | |
| RNAse-free water | | | | Add to 4 ml | | |
| Total: | | | | 4 ml | | |

In the present invention, the hybridization buffer may contain a higher SSC concentration - which would create a higher Na⁺ concentration - and a higher dextran sulfate concentration than normally used for RNA FISH, and also includes a high MgCl₂ concentration. Both serve to stabilize the probes as well as possible marker molecules such as a DNA origami marker molecule. These higher concentrations may be, but not limited to, exemplified as follows:

**Table 2.5 Hybridization buffer (2x) - Higher concentrations**

| | **Stock Concentration** | **Final concentration** |
|---|---|---|
| **Dextran sulfate** | 60% | 20% |
| **E. coli tRNA** | 40 mg/ml | 1,00 mg/mL |
| **Vanadyl Ribon. Complex** | 200 mM | 4 mM |
| **BSA (Ultra Pure)** | 50 mg/mL | 0,04 mg/mL |
| **SSC** | 20 x | 8 x |
| **Formamide, deionized** | 100% | 20 % |
| **MgCl₂** | 1000 mM | 22 mM |
| **RNAse-free water** | As needed | As needed |

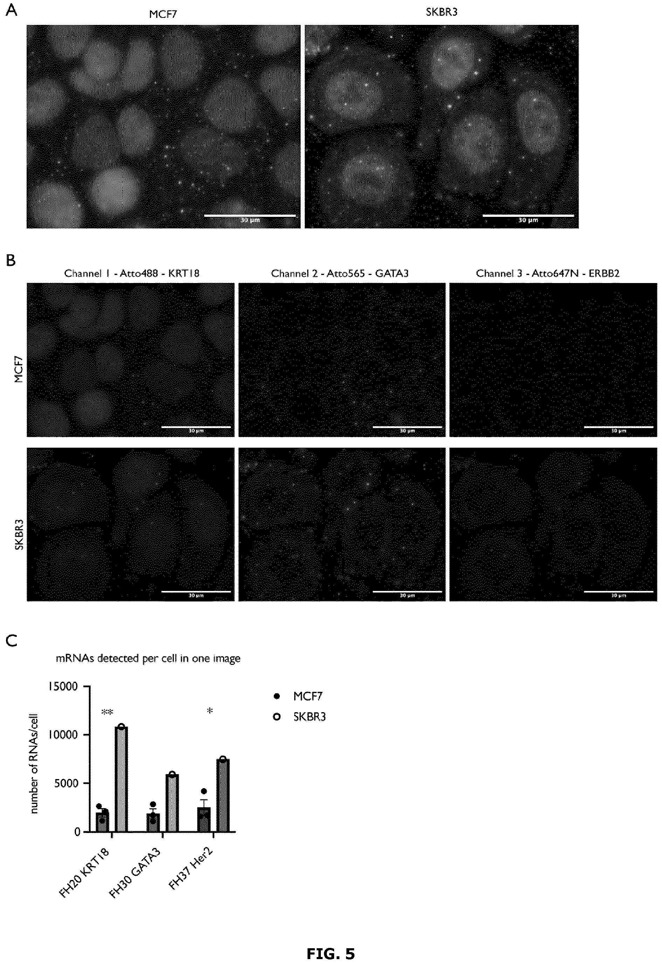

Fig. 5 depicts a multiplex detection of three cancer biomarkers (KRT18, GATA3 and ERBB2 (Her2/neu)) using single-color probe according to embodiments of the present inventions in breast cancer cell lines. Fig. 5A depicts an overlay images of nuclear Hoechst stain (blue), KRT18 mRNA (cyan), GATA3 mRNA (green) und ERBB2/Her2/neu mRNA (red) in the Her2/neu-wild type cell line MCF7, und the Her2/neu-overexpressing cell line SKBR3. Fig. 5B single channel images of the overlays shown in Fig. 5A. Fig. 5C depicts an absolute quantification of the detected RNAs using the open-source software, FISH-quant v2. Scale bar 30 µm. * p<0.05, ** p<0.01.

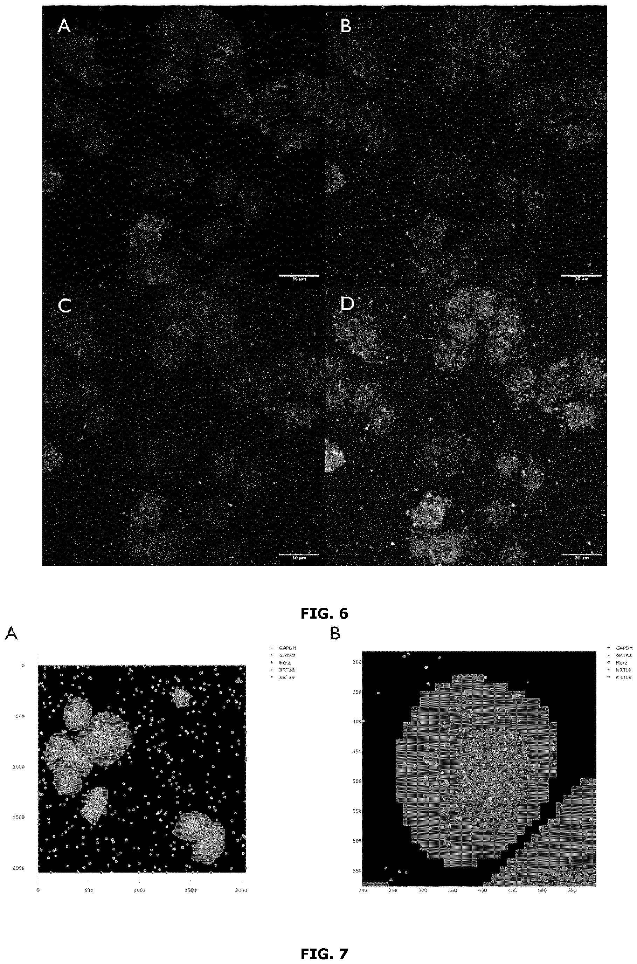

Fig. 6 depicts a multiplex detection of 5 cancer biomarker RNAs with one probe according to embodiments of the present invention each, containing one or two different types of fluorophores (KRT19 detected with Atto488 in cyan, GAPDH with Atto565 in green, ERBB2 with Atto565 and Atto647N in green and red, GATA3 with Atto488 and Atto565 in cyan and green, and KRT18 with Atto488 and Atto647N in cyan and red). Fig. 6A depicts a signal in the red channel detecting Atto647N. Fig. 6B depicts a signal in the green channel detecting Atto565. Fig. 6C depicts a signal in the cyan channel detecting Atto488. Fig. 6D Overlay image of Figs. 6A-6C with Hoechst nuclear stain (blue). Dual-color probe according to embodiments of the present inventions contain 50% of the fluorophore molecules per fluorophore type, compared to a single-color probe according to embodiments of the present invention. The signal of RNAs labelled with one probe according to embodiments of the present invention with two fluorophore types is extracted based on the intensity difference of that signal compared to the single-color signal.

Fig. 7 depicts images of an example of false-color labelling of multi-color probe according to embodiments of the present invention. Fig. 7A depicts an example microscopy image of the experiment portrayed in Figure 4. Each signal from one of the 5 used single- or dual-color probe according to embodiments of the present inventions has been assigned to and replaced by a false-color circle, which encodes the signal of a single RNA species. The legend on the right of the image gives the color code for assigning the false-color circles to the respective RNA species. Fig. 7B depicts a zoomed-in view of one cell from Fig. 7A.

The table below comprises a list of sequences according to embodiments of the present invention. Art. seq. refers to "artificial sequence"; St. str. refers "staple strand".

| **SEQ ID NO** | **LEN GTH** | **TYP E** | **ORGAN ISM** | **FEATURE** | **MORE INFO** | **SEQ.** |
|---|---|---|---|---|---|---|
| 1 | 28 | DNA | Art. seq. | St. str. for DNA nanostructure | | TTAAATCGCTGCTCGGCTGACTCTGGTC |
| 2 | 28 | DNA | Art. seq. | St. str. for DNA nanostructure | | GCGTTTTCAGAACGAAAGAGGGGTTATC |
| 3 | 28 | DNA | Art. seq. | St. str. for DNA nanostructure | | GTACCTTAACGTCAAACAACCCCATCAA |
| 4 | 28 | DNA | Art. seq. | St. str. for DNA nanostructure | | TGCTTTGACGTAAACCGTAATAAATTTT |
| 5 | 35 | DNA | Art. seq. | St. str. for DNA nanostructure | | AAATCTAAAACCACTTAATTTCTAGCATAAGGCTA |
| 6 | 35 | DNA | Art. seq. | St. str. for DNA nanostructure | | TTAAGAATCAGATGAACAATTGCCCCAAAAATTAA |
| 7 | 49 | DNA | Art. seq. | St. str. for DNA nanostructure | | GAGCCTATGTACTGAATCCTCATTAAAGCCTCAGACAAGCCGGTCCTGA |
| 8 | 49 | DNA | Art. seq. | St. str. for DNA nanostructure | | GAACAAGGCCGCCAGGTCATAGCCCCCTAGCAAAAGTACCGATTATACA |
| 9 | 49 | DNA | Art. seq. | St. str. for DNA nanostructure | | CATTCCAAGAGCCGGCCTTTAGCGTCAGAACGTCATTAGGCAGGGCTTA |
| 10 | 49 | DNA | Art. seq. | St. str. for DNA nanostructure | | ATTTTATCAGTGCCGAGGCAGGTCAGACCACCCTCAGAACGGATGTAGA |
| 11 | 56 | DNA | Art. seq. | St. str. for DNA nanostructure | | AGCAACGACAGCTTATATTACTCCATCATGCTGAATATAATGAAGTACGTAGTAGT |
| 12 | 56 | DNA | Art. seq. | St. str. for DNA nanostructure | | ATCGTCATCGCCCAGAAGAACAGTAATATTTTGATAAGAGGTCCCAATTCATTTGG |
| 13 | 28 | DNA | Art. seq. | St. str. for DNA nanostructure | | ATTAACAAAACATCACGGGTAAGGCTTT |
| 14 | 28 | DNA | Art. seq. | St. str. for DNA nanostructure | | AAATGAATTGAATGAACGAAACAGCGAA |
| 15 | 35 | DNA | Art. seq. | St. str. for DNA nanostructure | | AGATTTTAGCGGAAGACGTTGGAATAAGGAGGTTT |
| 16 | 35 | DNA | Art. seq. | St. str. for DNA nanostructure | | TCAAAATTTCATCATACCTTAAATTGGGTCTAAGA |
| 17 | 35 | DNA | Art. seq. | St. str. for DNA nanostructure | | TTTGCGTATTGGGCGTCGGGACCGTTCTGAGAAAG |
| 18 | 35 | DNA | Art. seq. | St. str. for DNA nanostructure | | AGACGGGCAACAGCCTAACTCTTGAGAGATGCAAT |
| 19 | 42 | DNA | Art. seq. | St. str. for DNA nanostructure | | AGAGTCCACTATTATCCCTTATTAAAATAAGATTGTATTAGA |
| 20 | 42 | DNA | Art. seq. | St. str. for DNA nanostructure | | GAAAAACCGTCTATAGGCGAAAACCAATATATGTATTGCCCG |
| 21 | 42 | DNA | Art. seq. | St. str. for DNA nanostructure | | ACCAAGTACCCTCACGCGTTTTCATCGGATTACCACCAGTAA |
| 22 | 42 | DNA | Art. seq. | St. str. for DNA nanostructure | | TTTCATCACCGGAAGTTTGCCATCTTTTAGCCATTATTCTGT |
| 23 | 28 | DNA | Art. seq. | St. str. for DNA nanostructure | | AAAACATATTTTAAATCTACAGTCAATC |
| 24 | 28 | DNA | Art. seq. | St. str. for DNA nanostructure | | AGCAATAAAAGGGTAGCTGATAAACAGG |
| 25 | 35 | DNA | Art. seq. | St. str. for DNA nanostructure | | ACAACCACCCTCAGGAGGCAAGTATCATATAGGCT |
| 26 | 35 | DNA | Art. seq. | St. str. for DNA nanostructure | | ATCCAATCGCAAGATTTAGTTAACAGTAGAGGCAT |
| 27 | 35 | DNA | Art. seq. | St. str. for DNA nanostructure | | TTCTTAAGCTACAGAAATACGCCATGTTAACTTTG |
| 28 | 35 | DNA | Art. seq. | St. str. for DNA nanostructure | | AGTTACAAACCCTCGGCATAGTAAGAGCTGTGAAT |
| 29 | 35 | DNA | Art. seq. | St. str. for DNA nanostructure | | GCTTAGGTTGGGTTATACCGAATATGCGCAAAAGG |
| 30 | 35 | DNA | Art. seq. | St. str. for DNA nanostructure | | GGGAGAAAGGCTTTCATTCAACTAATGCCAGTCAG |
| 31 | 56 | DNA | Art. seq. | St. str. for DNA nanostructure | | CTTAGTGGCAACTACTGTAGCAGTAAAAGAGTCTGCGCCAGCTGGCAGAAGCCCTA |
| 32 | 56 | DNA | Art. seq. | St. str. for DNA nanostructure | | TCTTCTAGTTGATTCATTTTTATACTTCTTTGATTTCAAACTTCTGGCCAATATTT |
| 33 | 28 | DNA | Art. seq. | St. str. for DNA nanostructure | | GTAAGAATGAACCTACCCTCATTGAGTA |
| 34 | 28 | DNA | Art. seq. | St. str. for DNA nanostructure | | GTCTTTATGAGAGCAAGGAATTCGTATT |
| 35 | 28 | DNA | Art. seq. | St. str. for DNA nanostructure | | AAAATACGTGAGGCAACAACTTGAGGAT |
| 36 | 35 | DNA | Art. seq. | St. str. for DNA nanostructure | | TAACCTGACTTTTGAAATTTTATTGCCTCGGTAAT |
| 37 | 35 | DNA | Art. seq. | St. str. for DNA nanostructure | | AGGTGGCAAAGCTATAATGTGGAGGGTATGATAAT |
| 38 | 35 | DNA | Art. seq. | St. str. for DNA nanostructure | | TATAGCCGGATTAGGATTAGCACAGGAGATTTGCC |
| 39 | 35 | DNA | Art. seq. | St. str. for DNA nanostructure | | GAAGTTTAGGCGCACATAAGGTGGTTTATAATCAT |
| 40 | 35 | DNA | Art. seq. | St. str. for DNA nanostructure | | ATAAATCGGCAAAGACAGTCAAATATGAAAATATT |
| 41 | 42 | DNA | Art. seq. | St. str. for DNA nanostructure | | TATATGTAAATGCTACCTAAAACCAGTATAAGAGAAAATAAT |
| 42 | 42 | DNA | Art. seq. | St. str. for DNA nanostructure | | AGAGCGGGGAGGCCTTATAATCAGTGAGAAACGCTTCTGAAA |
| 43 | 42 | DNA | Art. seq. | St. str. for DNA nanostructure | | CGCGAGAAATTCTGGAATTACATCGCCACGCCAACCAATAAT |
| 44 | 49 | DNA | Art. seq. | St. str. for DNA nanostructure | | TAGAAAATCTTCTGGATGCAATCCAAAAAAAAGGCATAGTTGATCGGAA |
| 45 | 49 | DNA | Art. seq. | St. str. for DNA nanostructure | | AGTAAATTATGGGAACTAAAGGAATTGCCCGATATGCCGCTTACACTCA |
| 46 | 49 | DNA | Art. seq. | St. str. for DNA nanostructure | | ACGCAAAATAAATAACCTCCGTTATCAGCTCGATACATAGTGAAAGACT |
| 47 | 56 | DNA | Art. seq. | St. str. for DNA nanostructure | | TTGAAAAGCTTAGAAGACTACCTTTTTAAGGCGTTAGAAAAACCAGACGAACGCGC |
| 48 | 28 | DNA | Art. seq. | St. str. for DNA nanostructure | | CTGATTACTGGCTCGAAACACAGCGAAC |
| 49 | 28 | DNA | Art. seq. | St. str. for DNA nanostructure | | AACAAAGCGTTAATTAACAAAAAGATTA |
| 50 | 35 | DNA | Art. seq. | St. str. for DNA nanostructure | | GCCTAATCCACACAACATACGCTGACGCCTGTAAT |
| 51 | 35 | DNA | Art. seq. | St. str. for DNA nanostructure | | CTGCCCGTCATAGCTGTTTCCCTGGTTGAGAGCAT |
| 52 | 35 | DNA | Art. seq. | St. str. for DNA nanostructure | | TACCGCCTGAAACATGAAAGTTGAGTAACCTGAAT |
| 53 | 49 | DNA | Art. seq. | St. str. for DNA nanostructure | | CAACGTCGTGGTTCTGTTAAACAGAAAACGACAACTGAGGAAACAGATG |
| 54 | 49 | DNA | Art. seq. | St. str. for DNA nanostructure | | CATGCGCGAAGTTTGCTTAATCGCAAATTAACCGTTAATATCGACCAGT |
| 55 | 49 | DNA | Art. seq. | St. str. for DNA nanostructure | | AGGAGAACGAGTAGTTGCTCCACATCACTTTCGCGAAATAGCCCTTCTG |
| 56 | 49 | DNA | Art. seq. | St. str. for DNA nanostructure | | CGAAATCTGGTTTTTCTTTTCGCGCTCATGCCGGATAGGTAAGCCACGC |
| 57 | 49 | DNA | Art. seq. | St. str. for DNA nanostructure | | ACTACGTTCCACGCAAATAATCGTAAAATAAAAGTATCAATACTTCATC |
| 58 | 49 | DNA | Art. seq. | St. str. for DNA nanostructure | | TGGTTTGCCTTCACCGCCTGGCTGGGGTTCAGGTCTAGAACCACCTTGC |
| 59 | 49 | DNA | Art. seq. | St. str. for DNA nanostructure | | AGCCACCGTAGGAACTGTTTACGGAACGCCATTAAGCCATTATGGATTA |
| 60 | 49 | DNA | Art. seq. | St. str. for DNA nanostructure | | AACCGCCACCGCACATCCCATTAAATTGAACCTAAGCTATTAAATAAAA |
| 61 | 56 | DNA | Art. seq. | St. str. for DNA nanostructure | | CGTCGAGTGCTCAGTACCAGGTCATACACAGCCATCCGGTATCTTGAGAGAACCGA |
| 62 | 56 | DNA | Art. seq. | St. str. for DNA nanostructure | | CACCGTAGGCTGAGACTCCTCGTTTTAACGAGCGTCTTGCGGGCTTGCCTACAGAC |
| 63 | 40 | DNA | Art. seq. | St. str. for DNA nanostructure | | CCCCGCTTTATGACAATGTCCCGAAAAAAAACCAAAATAA |
| 64 | 35 | DNA | Art. seq. | St. str. for DNA nanostructure | | CAGCGCCAATATATCAAAGAAAATTTTTTACGTT |
| 65 | 35 | DNA | Art. seq. | St. str. for DNA nanostructure | | CTCAGGAGGTTTAGAAGTCAGGAGAATTATTTAGG |
| 66 | 35 | DNA | Art. seq. | St. str. for DNA nanostructure | | AGTTTATGTTTGAAATATAACGGAGCCTTTAATTG |
| 67 | 35 | DNA | Art. seq. | St. str. for DNA nanostructure | | AGGGTTGATATAAGACAAGAATACAGAGAAAAGGA |
| 68 | 42 | DNA | Art. seq. | St. str. for DNA nanostructure | | ACCTGCTTAATGCCAACTGATTTCACCACTACTAAGTGTCTG |
| 69 | 42 | DNA | Art. seq. | St. str. for DNA nanostructure | | CAACAGTAGATTCAAAGCCTCGTGTAATGACTCTATGATACC |
| 70 | 42 | DNA | Art. seq. | St. str. for DNA nanostructure | | GAGATTTAAGAATACACAGACAACAGAGATATTTTCTGCGAA |
| 71 | 42 | DNA | Art. seq. | St. str. for DNA nanostructure | | AAGCATCCTCATATTATGACCATTTCACACTCGTCGGTGGGC |
| 72 | 35 | DNA | Art. seq. | St. str. for DNA nanostructure | | AGCGCATACGCTAACGGGGTCCTTGATATTCACAA |
| 73 | 35 | DNA | Art. seq. | St. str. for DNA nanostructure | | AAAATAGAAATAAATGGCTTTGGAAAGCGCAGTCT |
| 74 | 42 | DNA | Art. seq. | St. str. for DNA nanostructure | | TAGTATCCCGTGTGGACACCACGGAATAGTGAATTTAGAGCC |
| 75 | 42 | DNA | Art. seq. | St. str. for DNA nanostructure | | AACGCTCAATTTCATTCATATGGTTTACGAGGGAGGGCCGGA |
| 76 | 42 | DNA | Art. seq. | St. str. for DNA nanostructure | | TCTTTAGACTGACCACTTAGCTCAACAATAAAGTATGGGAAT |
| 77 | 49 | DNA | Art. seq. | St. str. for DNA nanostructure | | CAGGGCTAAAATTAAGCATTATTTACATCATTGCAGGTGAATTATCGGT |
| 78 | 49 | DNA | Art. seq. | St. str. for DNA nanostructure | | ATTTCTCTGTACCAGGCGCGAGGGACATATCGGCCAATGACAGAAAATC |
| 79 | 56 | DNA | Art. seq. | St. str. for DNA nanostructure | | AAATCAACAGGCGCCGCCTGACCTAATTTTTCGAGTTAGCAAGGAAGGTAAATATT |
| 80 | 23 | DNA | Art. seq. | St. str. for DNA nanostructure | | AAAAGTACGCCAGAATCCTGAGA |
| 81 | 23 | DNA | Art. seq. | St. str. for DNA nanostructure | | AAAAAAGGGAAGAAAGCGAAAGG |
| 82 | 23 | DNA | Art. seq. | St. str. for DNA nanostructure | | AAAAAGATGATGAAACAAACATC |
| 83 | 23 | DNA | Art. seq. | St. str. for DNA nanostructure | | AAAAAAGCAAACTCCAACAGGTC |
| 84 | 23 | DNA | Art. seq. | St. str. for DNA nanostructure | | AAAACGCCATTCGCCATTCAGGC |
| 85 | 23 | DNA | Art. seq. | St. str. for DNA nanostructure | | AAAAGCCCTTTTTAAGAAAAGTA |
| 86 | 23 | DNA | Art. seq. | St. str. for DNA nanostructure | | AAAAGAATTTATCAAAATCATAG |
| 87 | 23 | DNA | Art. seq. | St. str. for DNA nanostructure | | AAAACAGCGGAGTGAGAATAGAA |
| 88 | 23 | DNA | Art. seq. | St. str. for DNA nanostructure | | AAAACCACCACCCTCATTTTCAG |
| 89 | 23 | DNA | Art. seq. | St. str. for DNA nanostructure | | AAAAACTGCGGAATCGTCATAAA |
| 90 | 34 | DNA | Art. seq. | St. str. for DNA nanostructure | | TTTTGAAACTACAACGCCTAAAAAAAAGTAGCAT |
| 91 | 34 | DNA | Art. seq. | St. str. for DNA nanostructure | | ATGGTTGAAAAAAAACTTTGACGAGCACGGGCAA |
| 92 | 34 | DNA | Art. seq. | St. str. for DNA nanostructure | | ATTTCAACAGTACATAAATAAAAAAAACAATATA |
| 93 | 34 | DNA | Art. seq. | St. str. for DNA nanostructure | | TACCGATACCCAAAAGAACAAAAAAAATGGCATG |
| 94 | 39 | DNA | Art. seq. | St. str. for DNA nanostructure | | TCATTTGAATTAAAAAAAAACCTTTTTTAATGGAAACTT |
| 95 | 39 | DNA | Art. seq. | St. str. for DNA nanostructure | | AACCGAGGAAACAAAAAAAAGCAATAATAACGGATTCCA |
| 96 | 39 | DNA | Art. seq. | St. str. for DNA nanostructure | | GGCCTCTTCGCTAAAAAAAAATTACGCCAGCTGGTGCAG |
| 97 | 39 | DNA | Art. seq. | St. str. for DNA nanostructure | | AGAGTCGAAAGGGGGATGTAAAAAAAAGCTGCAAGGCGA |
| 98 | 39 | DNA | Art. seq. | St. str. for DNA nanostructure | | ATACATATAACGTGCTTTCAAAAAAAACTCGTTAGAATC |
| 99 | 39 | DNA | Art. seq. | St. str. for DNA nanostructure | | GTAACACTGAGTAAAAAAAATTCGTCACCAGTACCACCC |
| 100 | 40 | DNA | Art. seq. | St. str. for DNA nanostructure | | TCAATCGCATGGAAATACCTACAAAAAAAAATTTTGACGC |
| 101 | 40 | DNA | Art. seq. | St. str. for DNA nanostructure | | CCTGTAGCCAAAAAAAAAGCTTTCATCAACATTTGGCCTT |
| 102 | 40 | DNA | Art. seq. | St. str. for DNA nanostructure | | CAAGAACCGAAAAAAAAGATATTCATTACCCAAATCTTGA |
| 103 | 40 | DNA | Art. seq. | St. str. for DNA nanostructure | | TTTTGTTATCCCAATCCAAATAAAAAAAAAAGAAACGATT |
| 104 | 40 | DNA | Art. seq. | St. str. for DNA nanostructure | | TTAGATACAAAAAAAAATTTCGCAAATGGTCAATTGACCA |
| 105 | 40 | DNA | Art. seq. | St. str. for DNA nanostructure | | TCCAGTTAGCCCGAGATAGGGTTAAAAAAAAGAGTGTTGT |
| 106 | 40 | DNA | Art. seq. | St. str. for DNA nanostructure | | CGCTGAGGCAAAAAAAATTGCAGGGAGTTAAAGATTCGGT |
| 107 | 40 | DNA | Art. seq. | St. str. for DNA nanostructure | | AGCCTTTATAAAAAAAATTCAACGCAAGGATAACGGGAGA |
| 108 | 40 | DNA | Art. seq. | St. str. for DNA nanostructure | | AACAGAGCGAACGAACCACCAGCAAAAAAAAAGAAGATAA |
| 109 | 40 | DNA | Art. seq. | St. str. for DNA nanostructure | | GAACAACATAAAAAAAATATTACAGGTAGAAAGACTAACG |
| 110 | 40 | DNA | Art. seq. | St. str. for DNA nanostructure | | GAAGGGTGTTTGGATTATACTTCAAAAAAAATGAATAATG |
| 111 | 40 | DNA | Art. seq. | St. str. for DNA nanostructure | | TATAGAACGCGCCCAATAGCAAGAAAAAAAACAAATCAGA |
| 112 | 40 | DNA | Art. seq. | St. str. for DNA nanostructure | | AATTACTAAATAAGAATAAACACAAAAAAAACGGAATCAT |
| 113 | 40 | DNA | Art. seq. | St. str. for DNA nanostructure | | GGAACCTACAGTTAATGCCCCCTAAAAAAAAGCCTATTTC |
| 114 | 40 | DNA | Art. seq. | St. str. for DNA nanostructure | | ATCCCCGCTTGTTACCTCGATAAAAAAAAAAAGACGGAGG |
| 115 | 40 | DNA | Art. seq. | St. str. for DNA nanostructure | | GCGGGGAGCTGCATTAATGAATCAAAAAAAAGGCCAACGC |
| 116 | 40 | DNA | Art. seq. | St. str. for DNA nanostructure | | AATGCAGACGACAATAAACAACAAAAAAAAATGTTCAGCT |
| 117 | 40 | DNA | Art. seq. | St. str. for DNA nanostructure | | CCCCCAGCGAAAAAAAAATTATACCAAGCGCGATCTTTGA |
| 118 | 40 | DNA | Art. seq. | St. str. for DNA nanostructure | | ATACATTAATAGATTAGAGCCGTAAAAAAAACAATAGATA |
| 119 | 40 | DNA | Art. seq. | St. str. for DNA nanostructure | | TGGAGCAAAAAAAAAAACAAGAGAATCGATGAAGAGAGTC |
| 120 | 40 | DNA | Art. seq. | St. str. for DNA nanostructure | | ATTGACAAACCACCACCAGAGCCAAAAAAAAGCCGCCAGC |
| 121 | 40 | DNA | Art. seq. | St. str. for DNA nanostructure | | ACAGAATGATAGCAGCACCGTAAAAAAAAAATCAGTAGCG |
| 122 | 41 | DNA | Art. seq. | St. str. for DNA nanostructure | | TTAAAGTAGCATAAGTTGGGTAACGCAAAAAAAACAGGGTT |
| 123 | 41 | DNA | Art. seq. | St. str. for DNA nanostructure | | TAATCATACATTAATCAAAAATCAGGAAAAAAAATCTTTAC |
| 124 | 42 | DNA | Art. seq. | St. str. for DNA nanostructure | | AAACGTAGAATAAAGGTGAAAAAAAAGCAACATATAAAAGAA |
| 125 | 46 | DNA | Art. seq. | St. str. for DNA nanostructure | | TCCACAGGTCTTCGGCTACAGCCCTCATAGTAAAAAAAATAGCGTA |
| 126 | 46 | DNA | Art. seq. | St. str. for DNA nanostructure | | CTTAATGAAAAAAAACGCCGCTACAGGGCCCATCAATCAGCGTACT |
| 127 | 46 | DNA | Art. seq. | St. str. for DNA nanostructure | | TGTGAGTTCAATGACGGGAATAACCTTGCTTAAAAAAAACTGTAAA |
| 128 | 46 | DNA | Art. seq. | St. str. for DNA nanostructure | | CCTGACTATCAACAAATATTATAGTCAGAAGAAAAAAAACAAAGCG |
| 129 | 49 | DNA | Art. seq. | St. str. for DNA nanostructure | | ATTAAGACACCGAAAATCTCCTTATTACGCAAAAAAAAAGTATGTTAGC |
| 130 | 51 | DNA | Art. seq. | St. str. for DNA nanostructure | | TTCCCAGGCCTTTAGGGTCACGACGTTGTAAAAAAAAAAAACGACGGCCAG |
| 131 | 51 | DNA | Art. seq. | St. str. for DNA nanostructure | | AAAGACAAAAGAAAAAAAAGGCGAACCATCCAAGTTTCCACCAGGGAGGTT |
| 132 | 51 | DNA | Art. seq. | St. str. for DNA nanostructure | | GATTGCAAAAGCACCTGTCAAAAAGATTAAGAAAAAAAAAGGAAGCCCGAA |
| 133 | 51 | DNA | Art. seq. | St. str. for DNA nanostructure | | TCGTCGCATGAGTTTTCTATTAATTAATTTTAAAAAAAACCCTTAGAATCC |
| 134 | 51 | DNA | Art. seq. | St. str. for DNA nanostructure | | ACGATCTAACAATATTTAAAGTTTTGTCGTCAAAAAAAATTTCCAGACGTT |
| 135 | 51 | DNA | Art. seq. | St. str. for DNA nanostructure | | GTCACGCTGCGCAAAAAAAAGTAACCACCACACCTGTAATAAATCGCCGCG |
| 136 | 53 | DNA | Art. seq. | St. str. for DNA nanostructure | | ATAGTAATGAATCCCTTTAAACAGTTCAGAAAAAAAAAAAACGAGAATGACCA |
| 137 | 35 | DNA | Art. seq. | St. str. for DNA nanostructure | | ACTGTAGGAGCCACGATTGGCAGTGCCTATTAAGA |
| 138 | 35 | DNA | Art. seq. | St. str. for DNA nanostructure | | TATTAGCCCGCCTCCCAGAATTGATGATGGGGTTT |
| 139 | 35 | DNA | Art. seq. | St. str. for DNA nanostructure | | ATTCAGTGGAAGAAAATACCATGCAAAAGAAGTTT |
| 140 | 35 | DNA | Art. seq. | St. str. for DNA nanostructure | | AGTAGTATGCGATTATTACGAGTTTACCAGACGAC |
| 141 | 42 | DNA | Art. seq. | St. str. for DNA nanostructure | | AGGAACGCGTCGGAAGGAAGATCGCACTCAAATCAATGGCCC |
| 142 | 42 | DNA | Art. seq. | St. str. for DNA nanostructure | | AAATCTAAGTTGGCAACGTTACAGAAGGCAGGTTTTTACATC |
| 143 | 42 | DNA | Art. seq. | St. str. for DNA nanostructure | | TCGCATTGGGATAGGTGCATCTGCCAGTTCGGAACTGGACTC |
| 144 | 42 | DNA | Art. seq. | St. str. for DNA nanostructure | | CCGCCTGTAAAATACTTTACAATGGCAATATTTGCAATACCA |
| 145 | 42 | DNA | Art. seq. | St. str. for DNA nanostructure | | GGCAAAAAAGAACGCCTAAAGGGAGCCCCGTAACCGTCACGT |
| 146 | 42 | DNA | Art. seq. | St. str. for DNA nanostructure | | CCCCAGCCAGGGCGAGTTTTTTGGGGTCCGGCCTCTTCTCCG |
| 147 | 49 | DNA | Art. seq. | St. str. for DNA nanostructure | | ACGACTTAAGTGTCCTGGAGTGAGTAAAATCATGGCTTTCCAGCCAGGG |
| 148 | 49 | DNA | Art. seq. | St. str. for DNA nanostructure | | GCCAGGGTGGATGTGTAAGCAATAAATCCACAATTGAGTGAGTGATTGC |
| 149 | 49 | DNA | Art. seq. | St. str. for DNA nanostructure | | TACGAGCGTATTAATGAAGCCCTTACCATAGACGGAGGGTAATTGAGCG |
| 150 | 49 | DNA | Art. seq. | St. str. for DNA nanostructure | | TAGATAATTTTTATACGCGAGAGTTACACAGCCTTTTGAGTTAAGCCCA |
| 151 | 28 | DNA | Art. seq. | St. str. for DNA nanostructure | | TAGCACCCATTTTCCCCTCAGACAAATA |
| 152 | 35 | DNA | Art. seq. | St. str. for DNA nanostructure | | TGGAACAAGAGCTTGACGGGGGCTAGGGTGTAGCG |
| 153 | 35 | DNA | Art. seq. | St. str. for DNA nanostructure | | AGACTTCTTTTAATGAGAGTACCTTTAAATTTAGT |
| 154 | 35 | DNA | Art. seq. | St. str. for DNA nanostructure | | ATCACCGTCACCGACTCATACAAATATGCATAATC |
| 155 | 35 | DNA | Art. seq. | St. str. for DNA nanostructure | | ACGTTAAGAGCAAGCGCTGTGGGCGCATCCGATTT |
| 156 | 35 | DNA | Art. seq. | St. str. for DNA nanostructure | | CGTGGTGGGTACCGCGTGCCAGAGGCGGAAAGAAT |
| 157 | 35 | DNA | Art. seq. | St. str. for DNA nanostructure | | TCAGCTCAAACGGCGACGACGACAGTATGAGGTGC |
| 158 | 42 | DNA | Art. seq. | St. str. for DNA nanostructure | | CCTGATTTAGAACCGCAGAGGCGAATTACAAAATTAACAATT |
| 159 | 42 | DNA | Art. seq. | St. str. for DNA nanostructure | | CGTATAAATTATTCACCCTCAGAACCGCAAGCCCAATGTACC |
| 160 | 42 | DNA | Art. seq. | St. str. for DNA nanostructure | | TCGCGTCAAATGTGGCTTTCCGGCACCGCTGTTGGCGGTGCG |
| 161 | 42 | DNA | Art. seq. | St. str. for DNA nanostructure | | AGGCACCTGTCGAAAACGGTGCTGACGAATTATACAGATACA |
| 162 | 42 | DNA | Art. seq. | St. str. for DNA nanostructure | | TGCCAAGCTTTCTCACGAATAGAATCGGAGCCGGAGTAAAGC |
| 163 | 42 | DNA | Art. seq. | St. str. for DNA nanostructure | | TCCTTATGTTGCTAAGCTACACAAACCCATAGGCCCTGAACA |
| 164 | 42 | DNA | Art. seq. | St. str. for DNA nanostructure | | AACAAAGAAGAGTAATCAACGAAAACGAATTCATCGGGGGTA |
| 165 | 42 | DNA | Art. seq. | St. str. for DNA nanostructure | | TGACCTCTGTGTGATTGCGTTACCAGTGTTTGATGAAAGGGC |
| 166 | 42 | DNA | Art. seq. | St. str. for DNA nanostructure | | GTAATAAAAGAGAACGGAATAGGTGTATCTAATATACAGGGA |
| 167 | 42 | DNA | Art. seq. | St. str. for DNA nanostructure | | TCATCGACTCCCGACTTTCCACATAAAACAGAGAGATAACCC |
| 168 | 56 | DNA | Art. seq. | St. str. for DNA nanostructure | | GAACCAGCTGAATTGTAAGCGCGTTACCCAAAGGATAAGTGCATAATAAAAAAATG |
| 169 | 56 | DNA | Art. seq. | St. str. for DNA nanostructure | | TCATTACGGCTTATATTATTTTAACGTCGAGCAAGAAACAATAGCCGAAAGAAGGA |
| 170 | 35 | DNA | Art. seq. | St. str. for DNA nanostructure | | TTTAGCTATAGAACCTGAGTACGCATAAGAATAAT |
| 171 | 35 | DNA | Art. seq. | St. str. for DNA nanostructure | | TACGTGGCACTAAATTGCGGGATTGAGATTTTTCA |
| 172 | 35 | DNA | Art. seq. | St. str. for DNA nanostructure | | ATCAATTGTCACACCAGAACAGATACCGTCCAAAA |
| 173 | 35 | DNA | Art. seq. | St. str. for DNA nanostructure | | CGTAAAGCACTAAATTGAGGGGGATTGAACAGAAA |
| 174 | 42 | DNA | Art. seq. | St. str. for DNA nanostructure | | ATCCGCGACAAGAAATATAAATCACCAGATTATTCATTAAAG |
| 175 | 42 | DNA | Art. seq. | St. str. for DNA nanostructure | | TACAACGAACCAATATGTAATCCAATGAACATTCAACCGATT |
| 176 | 49 | DNA | Art. seq. | St. str. for DNA nanostructure | | CATATTCTAAAGAACGGATTCGCCTGATGATAAAATAACGCCAGAATAA |
| 177 | 49 | DNA | Art. seq. | St. str. for DNA nanostructure | | TTTGCGGATAAATGCCCTCACAGTAACATGCCAGAAGTTGAGAACTGAA |
| 178 | 56 | DNA | Art. seq. | St. str. for DNA nanostructure | | CCCTGAGCAAGCGGGAGCGATGAAGGACCATCACCCCAGCCAAGCGAGTGATGAAT |
| 179 | 56 | DNA | Art. seq. | St. str. for DNA nanostructure | | ATGCGCGACTACGACGAGGGTAATTCTTTTTAATGTTTGTCACAATCAAGACGGAA |
| 180 | 35 | DNA | Art. seq. | St. str. for DNA nanostructure | | GCTGAAATATAACAAGTGGTTGTGAATTGACAGTG |
| 181 | 35 | DNA | Art. seq. | St. str. for DNA nanostructure | | ACATCCATAAATATCTGAATTGTCAACCTCTAATC |
| 182 | 49 | DNA | Art. seq. | St. str. for DNA nanostructure | | ATATAATTTAGAAGTATAAGCTATTCAAAACCTGTAGCTCGAATTCGTA |
| 183 | 49 | DNA | Art. seq. | St. str. for DNA nanostructure | | TTATCATAAATCCTCCCCGGTGCTATTTACATTAAAATTGTTATCCGCT |
| 184 | 49 | DNA | Art. seq. | St. str. for DNA nanostructure | | ACCAAAATAGCGAGACAATAAATTGCGTTGGGAACATTTTTTAATCCTG |
| 185 | 49 | DNA | Art. seq. | St. str. for DNA nanostructure | | CAGTTGACAGCAGCGCCTGAGAATCGGTCGAACTCCGGCCCTGCCATCT |
| 186 | 49 | DNA | Art. seq. | St. str. for DNA nanostructure | | GAGCACTGGTCAGTGCCGGAGAATTAGCTAAGCTATATTTACGCTCGCC |
| 187 | 56 | DNA | Art. seq. | St. str. for DNA nanostructure | | TAAAGCCAGACAGCCGCCGACTTGCTGGTGTAGCAGCGGATGGCTTAGACATTCCA |
| 188 | 56 | DNA | Art. seq. | St. str. for DNA nanostructure | | GCCTGTTGAGGACTCTTTCGAACAGGAAGCCACCGTCAACATGTAAACAGAGCTTT |
| 189 | 56 | DNA | Art. seq. | St. str. for DNA nanostructure | | AACATTTAATTACACTATCATAAATCGCTACCATATGGTGTATATTTTGTAAATCA |
| 190 | 7560 | DNA | Art. seq. | Scaffold p7560 for DNA nanostructure (seq. by tilibit nanosystems GmbH) | | AGCTTGGCACTGGCCGTCGTTTTACAACGTCGTGACTGGGAAAACCCTGGCGTTAC CCAACTTAATCGCCTTGCAGCACATCCCCCTTTCGCCAGCTGGCGTAATAGCGAAGA GGCCCGCACCGATCGCCCTTCCCAACAGTTGCGCAGCCTGAATGGCGAATGGCGCT TTGCCTGGTTTCCGGCACCAGAAGCGGTGCCGGAAAGCTGGCTGGAGTGCGATCTT CCTGAGGCCGATACTGTCGTCGTCCCCTCAAACTGGCAGATGCACGGTTACGATGC GCCCATCTACACCAACGTGACCTATCCCATTACGGTCAATCCGCCGTTTGTTCCCAC GGAGAATCCGACGGGTTGTTACTCGCTCACATTTAATGTTGATGAAAGCTGGCTACA GGAAGGCCAGACGCGAATTATTTTTGATGGCGTTCCTATTGGTTAAAAAATGAGCTG ATTTAACAAAAATTTAATGCGAATTTTAACAAAATATTAACGTTTACAATTTAAATATT TGCTTATACAATCTTCCTGTTTTTGGGGCTTTTCTGATTATCAACCGGGGTACATATG ATTGACATGCTAGTTTTACGATTACCGTTCATCGATTCTCTTGTTTGCTCCAGACTCT CAGGCAATGACCTGATAGCCTTTGTAGATCTCTCAAAAATAGCTACCCTCTCCGGCA TTAATTTATCAGCTAGAACGGTTGAATATCATATTGATGGTGATTTGACTGTCTCCGG CCTTTCTCACCCTTTTGAATCTTTACCTACACATTACTCAGGCATTGCATTTAAAATAT ATGAGGGTTCTAAAAATTTTTATCCTTGCGTTGAAATAAAGGCTTCTCCCGCAAAAGT ATTACAGGGTCATAATGTTTTTGGTACAACCGATTTAGCTTTATGCTCTGAGGCTTTA TTGCTTAATTTTGCTAATTCTTTGCCTTGCCTGTATGATTTATTGGATGTTAATGCTAC TACTATTAGTAGAATTGATGCCACCTTTTCAGCTCGCGCCCCAAATGAAAATATAGCT AAACAGGTTATTGACCATTTGCGAAATGTATCTAATGGTCAAACTAAATCTACTCGTT CGCAGAATTGGGAATCAACTGTTATATGGAATGAAACTTCCAGACACCGTACTTTAG TTGCATATTTAAAACATGTTGAGCTACAGCATTATATTCAGCAATTAAGCTCTAAGCC ATCCGCAAAAATGACCTCTTATCAAAAGGAGCAATTAAAGGTACTCTCTAATCCTGA CCTGTTGGAGTTTGCTTCCGGTCTGGTTCGCTTTGAAGCTCGAATTAAAACGCGATA TTTGAAGTCTTTCGGGCTTCCTCTTAATCTTTTTGATGCAATCCGCTTTGCTTCTGAC TATAATAGTCAGGGTAAAGACCTGATTTTTGATTTATGGTCATTCTCGTTTTCTGAAC TGTTTAAAGCATTTGAGGGGGATTCAATGAATATTTATGACGATTCCGCAGTATTGG ACGCTATCCAGTCTAAACATTTTACTATTACCCCCTCTGGCAAAACTTCTTTTGCAAA AGCCTCTCGCTATTTTGGTTTTTATCGTCGTCTGGTAAACGAGGGTTATGATAGTGTT GCTCTTACTATGCCTCGTAATTCCTTTTGGCGTTATGTATCTGCATTAGTTGAATGTG GTATTCCTAAATCTCAACTGATGAATCTTTCTACCTGTAATAATGTTGTTCCGTTAGTT CGTTTTATTAACGTAGATTTTTCTTCCCAACGTCCTGACTGGTATAATGAGCCAGTTC TTAAAATCGCATAAGGTAATTCACAATGATTAAAGTTGAAATTAAACCATCTCAAGCC |
| | | | | | | CAATTTACTACTCGTTCTGGTGTTTCTCGTCAGGGCAAGCCTTATTCACTGAATGAGC AGCTTTGTTACGTTGATTTGGGTAATGAATATCCGGTTCTTGTCAAGATTACTCTTGA TGAAGGTCAGCCAGCCTATGCGCCTGGTCTGTACACCGTTCATCTGTCCTCTTTCAA AGTTGGTCAGTTCGGTTCCCTTATGATTGACCGTCTGCGCCTCGTTCCGGCTAAGTA ACATGGAGCAGGTCGCGGATTTCGACACAATTTATCAGGCGATGATACAAATCTCCG TTGTACTTTGTTTCGCGCTTGGTATAATCGCTGGGGGTCAAAGATGAGTGTTTTAGT GTATTCTTTTGCCTCTTTCGTTTTAGGTTGGTGCCTTCGTAGTGGCATTACGTATTTT ACCCGTTTAATGGAAACTTCCTCATGAAAAAGTCTTTAGTCCTCAAAGCCTCTGTAGC CGTTGCTACCCTCGTTCCGATGCTGTCTTTCGCTGCTGAGGGTGACGATCCCGCAAA AGCGGCCTTTAACTCCCTGCAAGCCTCAGCGACCGAATATATCGGTTATGCGTGGG CGATGGTTGTTGTCATTGTCGGCGCAACTATCGGTATCAAGCTGTTTAAGAAATTCA CCTCGAAAGCAAGCTGATAAACCGATACAATTAAAGGCTCCTTTTGGAGCCTTTTTTT TGGAGATTTTCAACGTGAAAAAATTATTATTCGCAATTCCTTTAGTTGTTCCTTTCTAT TCTCACTCCGCTGAAACTGTTGAAAGTTGTTTAGCAAAATCCCATACAGAAAATTCAT TTACTAACGTCTGGAAAGACGACAAAACTTTAGATCGTTACGCTAACTATGAGGGCT GTCTGTGGAATGCTACAGGCGTTGTAGTTTGTACTGGTGACGAAACTCAGTGTTACG GTACATGGGTTCCTATTGGGCTTGCTATCCCTGAAAATGAGGGTGGTGGCTCTGAG GGTGGCGGTTCTGAGGGTGGCGGTTCTGAGGGTGGCGGTACTAAACCTCCTGAGT ACGGTGATACACCTATTCCGGGCTATACTTATATCAACCCTCTCGACGGCACTTATC CGCCTGGTACTGAGCAAAACCCCGCTAATCCTAATCCTTCTCTTGAGGAGTCTCAGC CTCTTAATACTTTCATGTTTCAGAATAATAGGTTCCGAAATAGGCAGGGGGCATTAA CTGTTTATACGGGCACTGTTACTCAAGGCACTGACCCCGTTAAAACTTATTACCAGT ACACTCCTGTATCATCAAAAGCCATGTATGACGCTTACTGGAACGGTAAATTCAGAG ACTGCGCTTTCCATTCTGGCTTTAATGAGGATTTATTTGTTTGTGAATATCAAGGCCA ATCGTCTGACCTGCCTCAACCTCCTGTCAATGCTGGCGGCGGCTCTGGTGGTGGTT CTGGTGGCGGCTCTGAGGGTGGTGGCTCTGAGGGTGGCGGTTCTGAGGGTGGCGG CTCTGAGGGAGGCGGTTCCGGTGGTGGCTCTGGTTCCGGTGATTTTGATTATGAAA AGATGGCAAACGCTAATAAGGGGGCTATGACCGAAAATGCCGATGAAAACGCGCTA CAGTCTGACGCTAAAGGCAAACTTGATTCTGTCGCTACTGATTACGGTGCTGCTATC GATGGTTTCATTGGTGACGTTTCCGGCCTTGCTAATGGTAATGGTGCTACTGGTGAT TTTGCTGGCTCTAATTCCCAAATGGCTCAAGTCGGTGACGGTGATAATTCACCTTTA ATGAATAATTTCCGTCAATATTTACCTTCCCTCCCTCAATCGGTTGAATGTCGCCCTT TTGTCTTTGGCGCTGGTAAACCATATGAATTTTCTATTGATTGTGACAAAATAAACTT ATTCCGTGGTGTCTTTGCGTTTCTTTTATATGTTGCCACCTTTATGTATGTATTTTCTA CGTTTGCTAACATACTGCGTAATAAGGAGTCTTAATCATGCCAGTTCTTTTGGGTATT CCGTTATTATTGCGTTTCCTCGGTTTCCTTCTGGTAACTTTGTTCGGCTATCTGCTTA CTTTTCTTAAAAAGGGCTTCGGTAAGATAGCTATTGCTATTTCATTGTTTCTTGCTCTT ATTATTGGGCTTAACTCAATTCTTGTGGGTTATCTCTCTGATATTAGCGCTCAATTAC CCTCTGACTTTGTTCAGGGTGTTCAGTTAATTCTCCCGTCTAATGCGCTTCCCTGTTT TTATGTTATTCTCTCTGTAAAGGCTGCTATTTTCATTTTTGACGTTAAACAAAAAATCG TTTCTTATTTGGATTGGGATAAATAATATGGCTGTTTATTTTGTAACTGGCAAATTAG |
| | | | | | | GCTCTGGAAAGACGCTCGTTAGCGTTGGTAAGATTCAGGATAAAATTGTAGCTGGG TGCAAAATAGCAACTAATCTTGATTTAAGGCTTCAAAACCTCCCGCAAGTCGGGAGG TTCGCTAAAACGCCTCGCGTTCTTAGAATACCGGATAAGCCTTCTATATCTGATTTGC TTGCTATTGGGCGCGGTAATGATTCCTACGATGAAAATAAAAACGGCTTGCTTGTTC TCGATGAGTGCGGTACTTGGTTTAATACCCGTTCTTGGAATGATAAGGAAAGACAGC CGATTATTGATTGGTTTCTACATGCTCGTAAATTAGGATGGGATATTATTTTTCTTGT TCAGGACTTATCTATTGTTGATAAACAGGCGCGTTCTGCATTAGCTGAACATGTTGTT TATTGTCGTCGTCTGGACAGAATTACTTTACCTTTTGTCGGTACTTTATATTCTCTTAT TACTGGCTCGAAAATGCCTCTGCCTAAATTACATGTTGGCGTTGTTAAATATGGCGA TTCTCAATTAAGCCCTACTGTTGAGCGTTGGCTTTATACTGGTAAGAATTTGTATAAC GCATATGATACTAAACAGGCTTTTTCTAGTAATTATGATTCCGGTGTTTATTCTTATTT AACGCCTTATTTATCACACGGTCGGTATTTCAAACCATTAAATTTAGGTCAGAAGATG AAATTAACTAAAATATATTTGAAAAAGTTTTCTCGCGTTCTTTGTCTTGCGATTGGATT TGCATCAGCATTTACATATAGTTATATAACCCAACCTAAGCCGGAGGTTAAAAAGGT AGTCTCTCAGACCTATGATTTTGATAAATTCACTATTGACTCTTCTCAGCGTCTTAAT CTAAGCTATCGCTATGTTTTCAAGGATTCTAAGGGAAAATTAATTAATAGCGACGATT TACAGAAGCAAGGTTATTCACTCACATATATTGATTTATGTACTGTTTCCATTAAAAA AGGTAATTCAAATGAAATTGTTAAATGTAATTAATTTTGTTTTCTTGATGTTTGTTTCA TCATCTTCTTTTGCTCAGGTAATTGAAATGAATAATTCGCCTCTGCGCGATTTTGTAA CTTGGTATTCAAAGCAATCAGGCGAATCCGTTATTGTTTCTCCCGATGTAAAAGGTA CTGTTACTGTATATTCATCTGACGTTAAACCTGAAAATCTACGCAATTTCTTTATTTCT GTTTTACGTGCAAATAATTTTGATATGGTAGGTTCTAACCCTTCCATTATTCAGAAGT ATAATCCAAACAATCAGGATTATATTGATGAATTGCCATCATCTGATAATCAGGAATA TGATGATAATTCCGCTCCTTCTGGTGGTTTCTTTGTTCCGCAAAATGATAATGTTACT CAAACTTTTAAAATTAATAACGTTCGGGCAAAGGATTTAATACGAGTTGTCGAATTGT TTGTAAAGTCTAATACTTCTAAATCCTCAAATGTATTATCTATTGACGGCTCTAATCTA TTAGTTGTTAGTGCTCCTAAAGATATTTTAGATAACCTTCCTCAATTCCTTTCAACTGT TGATTTGCCAACTGACCAGATATTGATTGAGGGTTTGATATTTGAGGTTCAGCAAGG TGATGCTTTAGATTTTTCATTTGCTGCTGGCTCTCAGCGTGGCACTGTTGCAGGCGG TGTTAATACTGACCGCCTCACCTCTGTTTTATCTTCTGCTGGTGGTTCGTTCGGTATT TTTAATGGCGATGTTTTAGGGCTATCAGTTCGCGCATTAAAGACTAATAGCCATTCAA AAATATTGTCTGTGCCACGTATTCTTACGCTTTCAGGTCAGAAGGGTTCTATCTCTGT TGGCCAGAATGTCCCTTTTATTACTGGTCGTGTGACTGGTGAATCTGCCAATGTAAA TAATCCATTTCAGACGATTGAGCGTCAAAATGTAGGTATTTCCATGAGCGTTTTTCCT GTTGCAATGGCTGGCGGTAATATTGTTCTGGATATTACCAGCAAGGCCGATAGTTTG AGTTCTTCTACTCAGGCAAGTGATGTTATTACTAATCAAAGAAGTATTGCTACAACGG TTAATTTGCGTGATGGACAGACTCTTTTACTCGGTGGCCTCACTGATTATAAAAACAC TTCTCAGGATTCTGGCGTACCGTTCCTGTCTAAAATCCCTTTAATCGGCCTCCTGTTT AGCTCCCGCTCTGATTCTAACGAGGAAAGCACGTTATACGTGCTCGTCAAAGCAACC ATAGTACGCGCCCTGTAGCGGCGCATTAAGCGCGGCGGGTGTGGTGGTTACGCGC AGCGTGACCGCTACACTTGCCAGCGCCCTAGCGCCCGCTCCTTTCGCTTTCTTCCCT |
| | | | | | | TCCTTTCTCGCCACGTTCGCCGGCTTTCCCCGTCAAGCTCTAAATCGGGGGCTCCCT TTAGGGTTCCGATTTAGTGCTTTACGGCACCTCGACCCCAAAAAACTTGATTTGGGT GATGGTTCACGTAGTGGGCCATCGCCCTGATAGACGGTTTTTCGCCCTTTGACGTTG GAGTCCACGTTCTTTAATAGTGGACTCTTGTTCCAAACTGGAACAACACTCAACCCT ATCTCGGGCTATTCTTTTGATTTATAAGGGATTTTGCCGATTTCGGAACCACCATCAA ACAGGATTTTCGCCTGCTGGGGCAAACCAGCGTGGACCGCTTGCTGCAACTCTCTC AGGGCCAGGCGGTGAAGGGCAATCAGCTGTTGCCCGTCTCACTGGTGAAAAGAAAA ACCACCCTGGCGCCCAATACGCAAACCGCCTCTCCCCGCGCGTTGGCCGATTCATT AATGCAGCTGGCACGACAGGTTTCCCGACTGGAAAGCGGGCAGTGAGCGCAACGC AATTAATGTGAGTTAGCTCACTCATTAGGCACCCCAGGCTTTACACTTTATGCTTCCG GCTCGTATGTTGTGTGGAATTGTGAGCGGATAACAATTTCACACAGGAAACAGCTAT GACCATGATTACGAATTCGAGCTCGGTACCCGGGGATCCTCCGTCTTTATCGAGGTA ACAAGCACCACGTAGCTTAAGCCCTGTTTACTCATTACACCAACCAGGAGGTCAGAG TTCGGAGAAATGATTTATGTGAAATGCGTCAGCCGATTCAAGGCCCCTATATTCGTG CCCACCGACGAGTTGCTTACAGATGGCAGGGCCGCACTGTCGGTATCATAGAGTCA CTCCAGGGCGAGCGTAAATAGATTAGAAGCGGGGTTATTTTGGCGGGACATTGTCA TAAGGTTGACAATTCAGCACTAAGGACACTTAAGTCGTGCGCATGAATTCACAACCA CTTAGAAGAACATCCACCCTGGCTTCTCCTGAGAA |
| 191 | 64 | DNA | Human | p. seq. 1 | p. targ. ACTB v01 | TATTCATAATGTTTAGACTGGATAGCGTCCAATAAAAGATAGCACAGCCTGGATAGC AACGTAC |
| 192 | 64 | DNA | Human | p. seq. 2 | p. targ. ACTB v01 | AGGATTATCGAGCTTCAAAGCGAACCAGACCGGAAAAGATAGCACAGCCTGGATAG CAACGTAC |
| 193 | 64 | DNA | Human | p. seq. 3 | p. targ. ACTB v01 | TGCGCAACTTCTGGTGCCGGAAACCAGGCAAAGAAAAGATAGCACAGCCTGGATAG CAACGTAC |
| 194 | 64 | DNA | Human | p. seq. 4 | p. targ. ACTB v01 | AGGAACATTTTGCTAAACAACTTTCAACAGTTTAAAAGATAGCACAGCCTGGATAGCA ACGTAC |
| 195 | 64 | DNA | Human | p. seq. 5 | p. targ. ACTB v01 | GGATAGCCACCCTCAGAACCGCCACCCTCAGAGAAAAGATAGCACAGCCTGGATAG CAACGTAC |
| 196 | 64 | DNA | Human | p. seq. 6 | p. targ. ACTB v01 | AAGAAAATTCATTTCAATTACCTGAGCAAAAGAAAAAGATAGCACAGCCTGGATAGC AACGTAC |
| 197 | 64 | DNA | Human | p. seq. 7 | p. targ. ACTB v01 | AGCAGATGAAATAGCAATAGCTATCTTACCGAAAAAAGATAGCACAGCCTGGATAGC AACGTAC |
| 198 | 64 | DNA | Human | p. seq. 8 | p. targ. ACTB v01 | GTCTGAGTTAAGACGCTGAGAAGAGTCAATAGTAAAAGATAGCACAGCCTGGATAGC AACGTAC |
| 199 | 64 | DNA | Human | p. seq. 9 | p. targ. ACTB v01 | AGCGGGCAAAGCCGGCGAACGTGGCGAGAAAGGAAAAGATAGCACAGCCTGGATA GCAACGTAC |
| 200 | 64 | DNA | Human | p. seq. 10 | p. targ. ACTB v01 | AGTGTTTGATTAAAGGGATTTTAGACAGGAACGAAAAGATAGCACAGCCTGGATAGC AACGTAC |
| 201 | 67 | DNA | Human | p. seq. 1 | p. targ. ACTB v01 | TATTCATAATGTTTAGACTGGATAGCGTCCAATAAAACACCTTCTACAATGAGCTGCG TGTGGCTCC |
| 202 | 67 | DNA | Human | p. seq. 2 | p. targ. ACTB v01 | AGGATTATCGAGCTTCAAAGCGAACCAGACCGGAAAACACCTTCTACAATGAGCTGC GTGTGGCTCC |
| 203 | 67 | DNA | Human | p. seq. 3 | p. targ. ACTB v01 | TGCGCAACTTCTGGTGCCGGAAACCAGGCAAAGAAAACACCTTCTACAATGAGCTGC GTGTGGCTCC |
| 204 | 67 | DNA | Human | p. seq. 4 | p. targ. ACTB v01 | AGGAACATTTTGCTAAACAACTTTCAACAGTTTAAAACACCTTCTACAATGAGCTGCG TGTGGCTCC |
| 205 | 67 | DNA | Human | p. seq. 5 | p. targ. ACTB v01 | GGATAGCCACCCTCAGAACCGCCACCCTCAGAGAAAACACCTTCTACAATGAGCTGC GTGTGGCTCC |
| 206 | 67 | DNA | Human | p. seq. 6 | p. targ. ACTB v01 | AAGAAAATTCATTTCAATTACCTGAGCAAAAGAAAAACACCTTCTACAATGAGCTGCG TGTGGCTCC |
| 207 | 67 | DNA | Human | p. seq. 7 | p. targ. ACTB v01 | AGCAGATGAAATAGCAATAGCTATCTTACCGAAAAAACACCTTCTACAATGAGCTGC GTGTGGCTCC |
| 208 | 67 | DNA | Human | p. seq. 8 | p. targ. ACTB v01 | GTCTGAGTTAAGACGCTGAGAAGAGTCAATAGTAAAACACCTTCTACAATGAGCTGC GTGTGGCTCC |
| 209 | 67 | DNA | Human | p. seq. 9 | p. targ. ACTB v01 | AGCGGGCAAAGCCGGCGAACGTGGCGAGAAAGGAAAACACCTTCTACAATGAGCTG CGTGTGGCTCC |
| 210 | 67 | DNA | Human | p. seq. 10 | p. targ. ACTB v01 | AGTGTTTGATTAAAGGGATTTTAGACAGGAACGAAAACACCTTCTACAATGAGCTGC GTGTGGCTCC |
| 211 | 67 | DNA | Human | p. seq. 1 | p. targ. ACTB v03 | TATTCATAATGTTTAGACTGGATAGCGTCCAATAAAATTAATGTCACGCACGATTTCC CGCTCGGCC |
| 212 | 67 | DNA | Human | p. seq. 2 | p. targ. ACTB v03 | AGGATTATCGAGCTTCAAAGCGAACCAGACCGGAAAATTAATGTCACGCACGATTTC CCGCTCGGCC |
| 213 | 67 | DNA | Human | p. seq. 3 | p. targ. ACTB v03 | TGCGCAACTTCTGGTGCCGGAAACCAGGCAAAGAAAATTAATGTCACGCACGATTTC CCGCTCGGCC |
| 214 | 67 | DNA | Human | p. seq. 4 | p. targ. ACTB v03 | AGGAACATTTTGCTAAACAACTTTCAACAGTTTAAAATTAATGTCACGCACGATTTCC CGCTCGGCC |
| 215 | 67 | DNA | Human | p. seq. 5 | p. targ. ACTB v03 | GGATAGCCACCCTCAGAACCGCCACCCTCAGAGAAAATTAATGTCACGCACGATTTC CCGCTCGGCC |
| 216 | 67 | DNA | Human | p. seq. 6 | p. targ. ACTB v03 | AAGAAAATTCATTTCAATTACCTGAGCAAAAGAAAAATTAATGTCACGCACGATTTCC CGCTCGGCC |
| 217 | 67 | DNA | Human | p. seq. 7 | p. targ. ACTB v03 | AGCAGATGAAATAGCAATAGCTATCTTACCGAAAAAATTAATGTCACGCACGATTTCC CGCTCGGCC |
| 218 | 67 | DNA | Human | p. seq. 8 | p. targ. ACTB v03 | GTCTGAGTTAAGACGCTGAGAAGAGTCAATAGTAAAATTAATGTCACGCACGATTTC CCGCTCGGCC |
| 219 | 67 | DNA | Human | p. seq. 9 | p. targ. ACTB v03 | AGCGGGCAAAGCCGGCGAACGTGGCGAGAAAGGAAAATTAATGTCACGCACGATTT CCCGCTCGGCC |
| 220 | 67 | DNA | Human | p. seq. 10 | p. targ. ACTB v03 | AGTGTTTGATTAAAGGGATTTTAGACAGGAACGAAAATTAATGTCACGCACGATTTCC CGCTCGGCC |
| 221 | 67 | DNA | Human | p. seq. 1 | p. targ. EZR v01 | TATTCATAATGTTTAGACTGGATAGCGTCCAATAAAAGTGGTAACTCGGACATTGATT GGTTTCGGC |
| 222 | 67 | DNA | Human | p. seq. 2 | p. targ. EZR v01 | AGGATTATCGAGCTTCAAAGCGAACCAGACCGGAAAAGTGGTAACTCGGACATTGAT TGGTTTCGGC |
| 223 | 67 | DNA | Human | p. seq. 3 | p. targ. EZR v01 | TGCGCAACTTCTGGTGCCGGAAACCAGGCAAAGAAAAGTGGTAACTCGGACATTGA TTGGTTTCGGC |
| 224 | 67 | DNA | Human | p. seq. 4 | p. targ. EZR v01 | AGGAACATTTTGCTAAACAACTTTCAACAGTTTAAAAGTGGTAACTCGGACATTGATT GGTTTCGGC |
| 225 | 67 | DNA | Human | p. seq. 5 | p. targ. EZR v01 | GGATAGCCACCCTCAGAACCGCCACCCTCAGAGAAAAGTGGTAACTCGGACATTGAT TGGTTTCGGC |
| 226 | 67 | DNA | Human | p. seq. 6 | p. targ. EZR v01 | AAGAAAATTCATTTCAATTACCTGAGCAAAAGAAAAAGTGGTAACTCGGACATTGATT GGTTTCGGC |
| 227 | 67 | DNA | Human | p. seq. 7 | p. targ. EZR v01 | AGCAGATGAAATAGCAATAGCTATCTTACCGAAAAAAGTGGTAACTCGGACATTGAT TGGTTTCGGC |
| 228 | 67 | DNA | Human | p. seq. 8 | p. targ. EZR v01 | GTCTGAGTTAAGACGCTGAGAAGAGTCAATAGTAAAAGTGGTAACTCGGACATTGAT TGGTTTCGGC |
| 229 | 67 | DNA | Human | p. seq. 9 | p. targ. EZR v01 | AGCGGGCAAAGCCGGCGAACGTGGCGAGAAAGGAAAAGTGGTAACTCGGACATTG ATTGGTTTCGGC |
| 230 | 67 | DNA | Human | p. seq. 10 | p. targ. EZR v01 | AGTGTTTGATTAAAGGGATTTTAGACAGGAACGAAAAGTGGTAACTCGGACATTGAT TGGTTTCGGC |
| 231 | 67 | DNA | Human | p. seq. 1 | p. targ. EZR v02 | TATTCATAATGTTTAGACTGGATAGCGTCCAATAAAACCAGTTGTATTTGGCTGGATT GCAAACTCC |
| 232 | 67 | DNA | Human | p. seq. 2 | p. targ. EZR v02 | AGGATTATCGAGCTTCAAAGCGAACCAGACCGGAAAACCAGTTGTATTTGGCTGGAT TGCAAACTCC |
| 233 | 67 | DNA | Human | p. seq. 3 | p. targ. EZR v02 | TGCGCAACTTCTGGTGCCGGAAACCAGGCAAAGAAAACCAGTTGTATTTGGCTGGAT TGCAAACTCC |
| 234 | 67 | DNA | Human | p. seq. 4 | p. targ. EZR v02 | AGGAACATTTTGCTAAACAACTTTCAACAGTTTAAAACCAGTTGTATTTGGCTGGATT GCAAACTCC |
| 235 | 67 | DNA | Human | p. seq. 5 | p. targ. EZR v02 | GGATAGCCACCCTCAGAACCGCCACCCTCAGAGAAAACCAGTTGTATTTGGCTGGAT TGCAAACTCC |
| 236 | 67 | DNA | Human | p. seq. 6 | p. targ. EZR v02 | AAGAAAATTCATTTCAATTACCTGAGCAAAAGAAAAACCAGTTGTATTTGGCTGGATT GCAAACTCC |
| 237 | 67 | DNA | Human | p. seq. 7 | p. targ. EZR v02 | AGCAGATGAAATAGCAATAGCTATCTTACCGAAAAAACCAGTTGTATTTGGCTGGATT GCAAACTCC |
| 238 | 67 | DNA | Human | p. seq. 8 | p. targ. EZR v02 | GTCTGAGTTAAGACGCTGAGAAGAGTCAATAGTAAAACCAGTTGTATTTGGCTGGAT TGCAAACTCC |
| 239 | 67 | DNA | Human | p. seq. 9 | p. targ. EZR v02 | AGCGGGCAAAGCCGGCGAACGTGGCGAGAAAGGAAAACCAGTTGTATTTGGCTGGA TTGCAAACTCC |
| 240 | 67 | DNA | Human | p. seq. 10 | p. targ. EZR v02 | AGTGTTTGATTAAAGGGATTTTAGACAGGAACGAAAACCAGTTGTATTTGGCTGGAT TGCAAACTCC |
| 241 | 67 | DNA | Human | p. seq. 1 | p. targ. EZR v03 | TATTCATAATGTTTAGACTGGATAGCGTCCAATAAAACTGCGGCGCATATACAACTCA TGGTTGCCC |
| 242 | 67 | DNA | Human | p. seq. 2 | p. targ. EZR v03 | AGGATTATCGAGCTTCAAAGCGAACCAGACCGGAAAACTGCGGCGCATATACAACTC ATGGTTGCCC |
| 243 | 67 | DNA | Human | p. seq. 3 | p. targ. EZR v03 | TGCGCAACTTCTGGTGCCGGAAACCAGGCAAAGAAAACTGCGGCGCATATACAACT CATGGTTGCCC |
| 244 | 67 | DNA | Human | p. seq. 4 | p. targ. EZR v03 | AGGAACATTTTGCTAAACAACTTTCAACAGTTTAAAACTGCGGCGCATATACAACTCA TGGTTGCCC |
| 245 | 67 | DNA | Human | p. seq. 5 | p. targ. EZR v03 | GGATAGCCACCCTCAGAACCGCCACCCTCAGAGAAAACTGCGGCGCATATACAACTC ATGGTTGCCC |
| 246 | 67 | DNA | Human | p. seq. 6 | p. targ. EZR v03 | AAGAAAATTCATTTCAATTACCTGAGCAAAAGAAAAACTGCGGCGCATATACAACTCA TGGTTGCCC |
| 247 | 67 | DNA | Human | p. seq. 7 | p. targ. EZR v03 | AGCAGATGAAATAGCAATAGCTATCTTACCGAAAAAACTGCGGCGCATATACAACTC ATGGTTGCCC |
| 248 | 67 | DNA | Human | p. seq. 8 | p. targ. EZR v03 | GTCTGAGTTAAGACGCTGAGAAGAGTCAATAGTAAAACTGCGGCGCATATACAACTC ATGGTTGCCC |
| 249 | 67 | DNA | Human | p. seq. 9 | p. targ. EZR v03 | AGCGGGCAAAGCCGGCGAACGTGGCGAGAAAGGAAAACTGCGGCGCATATACAAC TCATGGTTGCCC |
| 250 | 67 | DNA | Human | p. seq. 10 | p. targ. EZR v03 | AGTGTTTGATTAAAGGGATTTTAGACAGGAACGAAAACTGCGGCGCATATACAACTC ATGGTTGCCC |
| 251 | 67 | DNA | Human | p. seq. 1 | p. targ. GATA3 v01 | TATTCATAATGTTTAGACTGGATAGCGTCCAATAAAAAATATTGTGAAGCTTGTAGTA GAGCCCACA |
| 252 | 67 | DNA | Human | p. seq. 2 | p. targ. GATA3 v01 | AGGATTATCGAGCTTCAAAGCGAACCAGACCGGAAAAAATATTGTGAAGCTTGTAGT AGAGCCCACA |
| 253 | 67 | DNA | Human | p. seq. 3 | p. targ. GATA3 v01 | TGCGCAACTTCTGGTGCCGGAAACCAGGCAAAGAAAAAATATTGTGAAGCTTGTAGT AGAGCCCACA |
| 254 | 67 | DNA | Human | p. seq. 4 | p. targ. GATA3 v01 | AGGAACATTTTGCTAAACAACTTTCAACAGTTTAAAAAATATTGTGAAGCTTGTAGTA GAGCCCACA |
| 255 | 67 | DNA | Human | p. seq. 5 | p. targ. GATA3 v01 | GGATAGCCACCCTCAGAACCGCCACCCTCAGAGAAAAAATATTGTGAAGCTTGTAGT AGAGCCCACA |
| 256 | 67 | DNA | Human | p. seq. 6 | p. targ. GATA3 v01 | AAGAAAATTCATTTCAATTACCTGAGCAAAAGAAAAAAATATTGTGAAGCTTGTAGTA GAGCCCACA |
| 257 | 67 | DNA | Human | p. seq. 7 | p. targ. GATA3 v01 | AGCAGATGAAATAGCAATAGCTATCTTACCGAAAAAAAATATTGTGAAGCTTGTAGTA GAGCCCACA |
| 258 | 67 | DNA | Human | p. seq. 8 | p. targ. GATA3 v01 | GTCTGAGTTAAGACGCTGAGAAGAGTCAATAGTAAAAAATATTGTGAAGCTTGTAGT AGAGCCCACA |
| 259 | 67 | DNA | Human | p. seq. 9 | p. targ. GATA3 v01 | AGCGGGCAAAGCCGGCGAACGTGGCGAGAAAGGAAAAAATATTGTGAAGCTTGTAG TAGAGCCCACA |
| 260 | 67 | DNA | Human | p. seq. 10 | p. targ. GATA3 v01 | AGTGTTTGATTAAAGGGATTTTAGACAGGAACGAAAAAATATTGTGAAGCTTGTAGT AGAGCCCACA |
| 261 | 67 | DNA | Human | p. seq. 1 | p. targ. GATA3 v02 | TATTCATAATGTTTAGACTGGATAGCGTCCAATAAAATGGATTTGCTAGACATTTTTC GGTTTCTGG |
| 262 | 67 | DNA | Human | p. seq. 2 | p. targ. GATA3 v02 | AGGATTATCGAGCTTCAAAGCGAACCAGACCGGAAAATGGATTTGCTAGACATTTTT CGGTTTCTGG |
| 263 | 67 | DNA | Human | p. seq. 3 | p. targ. GATA3 v02 | TGCGCAACTTCTGGTGCCGGAAACCAGGCAAAGAAAATGGATTTGCTAGACATTTTT CGGTTTCTGG |
| 264 | 67 | DNA | Human | p. seq. 4 | p. targ. GATA3 v02 | AGGAACATTTTGCTAAACAACTTTCAACAGTTTAAAATGGATTTGCTAGACATTTTTC GGTTTCTGG |
| 265 | 67 | DNA | Human | p. seq. 5 | p. targ. GATA3 v02 | GGATAGCCACCCTCAGAACCGCCACCCTCAGAGAAAATGGATTTGCTAGACATTTTT CGGTTTCTGG |
| 266 | 67 | DNA | Human | p. seq. 6 | p. targ. GATA3 v02 | AAGAAAATTCATTTCAATTACCTGAGCAAAAGAAAAATGGATTTGCTAGACATTTTTC GGTTTCTGG |
| 267 | 67 | DNA | Human | p. seq. 7 | p. targ. GATA3 v02 | AGCAGATGAAATAGCAATAGCTATCTTACCGAAAAAATGGATTTGCTAGACATTTTTC GGTTTCTGG |
| 268 | 67 | DNA | Human | p. seq. 8 | p. targ. GATA3 v02 | GTCTGAGTTAAGACGCTGAGAAGAGTCAATAGTAAAATGGATTTGCTAGACATTTTTC GGTTTCTGG |
| 269 | 67 | DNA | Human | p. seq. 9 | p. targ. GATA3 v02 | AGCGGGCAAAGCCGGCGAACGTGGCGAGAAAGGAAAATGGATTTGCTAGACATTTT TCGGTTTCTGG |
| 270 | 67 | DNA | Human | p. seq. 10 | p. targ. GATA3 v02 | AGTGTTTGATTAAAGGGATTTTAGACAGGAACGAAAATGGATTTGCTAGACATTTTTC GGTTTCTGG |
| 271 | 67 | DNA | Human | p. seq. 1 | p. targ. ESR1 v01 | TATTCATAATGTTTAGACTGGATAGCGTCCAATAAAACTCCTGTTTTTATCAATGGTG CACTGGTTG |
| 272 | 67 | DNA | Human | p. seq. 2 | p. targ. ESR1 v01 | AGGATTATCGAGCTTCAAAGCGAACCAGACCGGAAAACTCCTGTTTTTATCAATGGT GCACTGGTTG |
| 273 | 67 | DNA | Human | p. seq. 3 | p. targ. ESR1 v01 | TGCGCAACTTCTGGTGCCGGAAACCAGGCAAAGAAAACTCCTGTTTTTATCAATGGT GCACTGGTTG |
| 274 | 67 | DNA | Human | p. seq. 4 | p. targ. ESR1 v01 | AGGAACATTTTGCTAAACAACTTTCAACAGTTTAAAACTCCTGTTTTTATCAATGGTGC ACTGGTTG |
| 275 | 67 | DNA | Human | p. seq. 5 | p. targ. ESR1 v01 | GGATAGCCACCCTCAGAACCGCCACCCTCAGAGAAAACTCCTGTTTTTATCAATGGT GCACTGGTTG |
| 276 | 67 | DNA | Human | p. seq. 6 | p. targ. ESR1 v01 | AAGAAAATTCATTTCAATTACCTGAGCAAAAGAAAAACTCCTGTTTTTATCAATGGTG CACTGGTTG |
| 277 | 67 | DNA | Human | p. seq. 7 | p. targ. ESR1 v01 | AGCAGATGAAATAGCAATAGCTATCTTACCGAAAAAACTCCTGTTTTTATCAATGGTG CACTGGTTG |
| 278 | 67 | DNA | Human | p. seq. 8 | p. targ. ESR1 v01 | GTCTGAGTTAAGACGCTGAGAAGAGTCAATAGTAAAACTCCTGTTTTTATCAATGGTG CACTGGTTG |
| 279 | 67 | DNA | Human | p. seq. 9 | p. targ. ESR1 v01 | AGCGGGCAAAGCCGGCGAACGTGGCGAGAAAGGAAAACTCCTGTTTTTATCAATGG TGCACTGGTTG |
| 280 | 67 | DNA | Human | p. seq. 10 | p. targ. ESR1 v01 | AGTGTTTGATTAAAGGGATTTTAGACAGGAACGAAAACTCCTGTTTTTATCAATGGTG CACTGGTTG |
| 281 | 67 | DNA | Human | p. seq. 1 | p. targ. ESR1 v02 | TATTCATAATGTTTAGACTGGATAGCGTCCAATAAAACGATTATCTGAATTTGGCCTG TAGAATGCC |
| 282 | 67 | DNA | Human | p. seq. 2 | p. targ. ESR1 v02 | AGGATTATCGAGCTTCAAAGCGAACCAGACCGGAAAACGATTATCTGAATTTGGCCT GTAGAATGCC |
| 283 | 67 | DNA | Human | p. seq. 3 | p. targ. ESR1 v02 | TGCGCAACTTCTGGTGCCGGAAACCAGGCAAAGAAAACGATTATCTGAATTTGGCCT GTAGAATGCC |
| 284 | 67 | DNA | Human | p. seq. 4 | p. targ. ESR1 v02 | AGGAACATTTTGCTAAACAACTTTCAACAGTTTAAAACGATTATCTGAATTTGGCCTG TAGAATGCC |
| 285 | 67 | DNA | Human | p. seq. 5 | p. targ. ESR1 v02 | GGATAGCCACCCTCAGAACCGCCACCCTCAGAGAAAACGATTATCTGAATTTGGCCT GTAGAATGCC |
| 286 | 67 | DNA | Human | p. seq. 6 | p. targ. ESR1 v02 | AAGAAAATTCATTTCAATTACCTGAGCAAAAGAAAAACGATTATCTGAATTTGGCCTG TAGAATGCC |
| 287 | 67 | DNA | Human | p. seq. 7 | p. targ. ESR1 v02 | AGCAGATGAAATAGCAATAGCTATCTTACCGAAAAAACGATTATCTGAATTTGGCCTG TAGAATGCC |
| 288 | 67 | DNA | Human | p. seq. 8 | p. targ. ESR1 v02 | GTCTGAGTTAAGACGCTGAGAAGAGTCAATAGTAAAACGATTATCTGAATTTGGCCT GTAGAATGCC |
| 289 | 67 | DNA | Human | p. seq. 9 | p. targ. ESR1 v02 | AGCGGGCAAAGCCGGCGAACGTGGCGAGAAAGGAAAACGATTATCTGAATTTGGCC TGTAGAATGCC |
| 290 | 67 | DNA | Human | p. seq. 10 | p. targ. ESR1 v02 | AGTGTTTGATTAAAGGGATTTTAGACAGGAACGAAAACGATTATCTGAATTTGGCCT GTAGAATGCC |
| 291 | 67 | DNA | Human | p. seq. 1 | p. targ. GAPDH v01 | TATTCATAATGTTTAGACTGGATAGCGTCCAATAAAACTTCTTGATGTCATCATATTTG GCAGGTTT |
| 292 | 67 | DNA | Human | p. seq. 2 | p. targ. GAPDH v01 | AGGATTATCGAGCTTCAAAGCGAACCAGACCGGAAAACTTCTTGATGTCATCATATTT GGCAGGTTT |
| 293 | 67 | DNA | Human | p. seq. 3 | p. targ. GAPDH v01 | TGCGCAACTTCTGGTGCCGGAAACCAGGCAAAGAAAACTTCTTGATGTCATCATATT TGGCAGGTTT |
| 294 | 67 | DNA | Human | p. seq. 4 | p. targ. GAPDH v01 | AGGAACATTTTGCTAAACAACTTTCAACAGTTTAAAACTTCTTGATGTCATCATATTTG GCAGGTTT |
| 295 | 67 | DNA | Human | p. seq. 5 | p. targ. GAPDH v01 | GGATAGCCACCCTCAGAACCGCCACCCTCAGAGAAAACTTCTTGATGTCATCATATTT GGCAGGTTT |
| 296 | 67 | DNA | Human | p. seq. 6 | p. targ. GAPDH v01 | AAGAAAATTCATTTCAATTACCTGAGCAAAAGAAAAACTTCTTGATGTCATCATATTTG GCAGGTTT |
| 297 | 67 | DNA | Human | p. seq. 7 | p. targ. GAPDH v01 | AGCAGATGAAATAGCAATAGCTATCTTACCGAAAAAACTTCTTGATGTCATCATATTT GGCAGGTTT |
| 298 | 67 | DNA | Human | p. seq. 8 | p. targ. GAPDH v01 | GTCTGAGTTAAGACGCTGAGAAGAGTCAATAGTAAAACTTCTTGATGTCATCATATTT GGCAGGTTT |
| 299 | 67 | DNA | Human | p. seq. 9 | p. targ. GAPDH v01 | AGCGGGCAAAGCCGGCGAACGTGGCGAGAAAGGAAAACTTCTTGATGTCATCATAT TTGGCAGGTTT |
| 300 | 67 | DNA | Human | p. seq. 10 | p. targ. GAPDH v02 | AGTGTTTGATTAAAGGGATTTTAGACAGGAACGAAAACTTCTTGATGTCATCATATTT GGCAGGTTT |
| 301 | 67 | DNA | Human | p. seq. 1 | p. targ. GAPDH v02 | TATTCATAATGTTTAGACTGGATAGCGTCCAATAAAATCGCTGTTGAAGTCAGAGGA GACCACCTGG |
| 302 | 67 | DNA | Human | p. seq. 2 | p. targ. GAPDH v02 | AGGATTATCGAGCTTCAAAGCGAACCAGACCGGAAAATCGCTGTTGAAGTCAGAGG AGACCACCTGG |
| 303 | 67 | DNA | Human | p. seq. 3 | p. targ. GAPDH v02 | TGCGCAACTTCTGGTGCCGGAAACCAGGCAAAGAAAATCGCTGTTGAAGTCAGAGG AGACCACCTGG |
| 304 | 67 | DNA | Human | p. seq. 4 | p. targ. GAPDH v02 | AGGAACATTTTGCTAAACAACTTTCAACAGTTTAAAATCGCTGTTGAAGTCAGAGGAG ACCACCTGG |
| 305 | 67 | DNA | Human | p. seq. 5 | p. targ. GAPDH v02 | GGATAGCCACCCTCAGAACCGCCACCCTCAGAGAAAATCGCTGTTGAAGTCAGAGG AGACCACCTGG |
| 306 | 67 | DNA | Human | p. seq. 6 | p. targ. GAPDH v02 | AAGAAAATTCATTTCAATTACCTGAGCAAAAGAAAAATCGCTGTTGAAGTCAGAGGA GACCACCTGG |
| 307 | 67 | DNA | Human | p. seq. 7 | p. targ. GAPDH v02 | AGCAGATGAAATAGCAATAGCTATCTTACCGAAAAAATCGCTGTTGAAGTCAGAGGA GACCACCTGG |
| 308 | 67 | DNA | Human | p. seq. 8 | p. targ. GAPDH v02 | GTCTGAGTTAAGACGCTGAGAAGAGTCAATAGTAAAATCGCTGTTGAAGTCAGAGGA GACCACCTGG |
| 309 | 67 | DNA | Human | p. seq. 9 | p. targ. GAPDH v02 | AGCGGGCAAAGCCGGCGAACGTGGCGAGAAAGGAAAATCGCTGTTGAAGTCAGAG GAGACCACCTGG |
| 310 | 67 | DNA | Human | p. seq. 10 | p. targ. GAPDH v02 | AGTGTTTGATTAAAGGGATTTTAGACAGGAACGAAAATCGCTGTTGAAGTCAGAGGA GACCACCTGG |
| 311 | 67 | DNA | Human | p. seq. 1 | p. targ. KRT18 v01 | TATTCATAATGTTTAGACTGGATAGCGTCCAATAAAATCCAAGGCATCACCAAGATTA AAGTCCTCG |
| 312 | 67 | DNA | Human | p. seq. 2 | p. targ. KRT18 v01 | AGGATTATCGAGCTTCAAAGCGAACCAGACCGGAAAATCCAAGGCATCACCAAGATT AAAGTCCTCG |
| 313 | 67 | DNA | Human | p. seq. 3 | p. targ. KRT18 v01 | TGCGCAACTTCTGGTGCCGGAAACCAGGCAAAGAAAATCCAAGGCATCACCAAGATT AAAGTCCTCG |
| 314 | 67 | DNA | Human | p. seq. 4 | p. targ. KRT18 v01 | AGGAACATTTTGCTAAACAACTTTCAACAGTTTAAAATCCAAGGCATCACCAAGATTA AAGTCCTCG |
| 315 | 67 | DNA | Human | p. seq. 5 | p. targ. KRT18 v01 | GGATAGCCACCCTCAGAACCGCCACCCTCAGAGAAAATCCAAGGCATCACCAAGATT AAAGTCCTCG |
| 316 | 67 | DNA | Human | p. seq. 6 | p. targ. KRT18 v01 | AAGAAAATTCATTTCAATTACCTGAGCAAAAGAAAAATCCAAGGCATCACCAAGATTA AAGTCCTCG |
| 317 | 67 | DNA | Human | p. seq. 7 | p. targ. KRT18 v01 | AGCAGATGAAATAGCAATAGCTATCTTACCGAAAAAATCCAAGGCATCACCAAGATT AAAGTCCTCG |
| 318 | 67 | DNA | Human | p. seq. 8 | p. targ. KRT18 v01 | GTCTGAGTTAAGACGCTGAGAAGAGTCAATAGTAAAATCCAAGGCATCACCAAGATT AAAGTCCTCG |
| 319 | 67 | DNA | Human | p. seq. 9 | p. targ. KRT18 v01 | AGCGGGCAAAGCCGGCGAACGTGGCGAGAAAGGAAAATCCAAGGCATCACCAAGA TTAAAGTCCTCG |
| 320 | 67 | DNA | Human | p. seq. 10 | p. targ. KRT18 v01 | AGTGTTTGATTAAAGGGATTTTAGACAGGAACGAAAATCCAAGGCATCACCAAGATT AAAGTCCTCG |
| 321 | 67 | DNA | Human | p. seq. 1 | p. targ. KRT18 v02 | TATTCATAATGTTTAGACTGGATAGCGTCCAATAAAATCGTGTGATATTGGTGTCATC AATGACCTT |
| 322 | 67 | DNA | Human | p. seq. 2 | p. targ. KRT18 v02 | AGGATTATCGAGCTTCAAAGCGAACCAGACCGGAAAATCGTGTGATATTGGTGTCAT CAATGACCTT |
| 323 | 67 | DNA | Human | p. seq. 3 | p. targ. KRT18 v02 | TGCGCAACTTCTGGTGCCGGAAACCAGGCAAAGAAAATCGTGTGATATTGGTGTCAT CAATGACCTT |
| 324 | 67 | DNA | Human | p. seq. 4 | p. targ. KRT18 v02 | AGGAACATTTTGCTAAACAACTTTCAACAGTTTAAAATCGTGTGATATTGGTGTCATC AATGACCTT |
| 325 | 67 | DNA | Human | p. seq. 5 | p. targ. KRT18 v02 | GGATAGCCACCCTCAGAACCGCCACCCTCAGAGAAAATCGTGTGATATTGGTGTCAT CAATGACCTT |
| 326 | 67 | DNA | Human | p. seq. 6 | p. targ. KRT18 v02 | AAGAAAATTCATTTCAATTACCTGAGCAAAAGAAAAATCGTGTGATATTGGTGTCATC AATGACCTT |
| 327 | 67 | DNA | Human | p. seq. 7 | p. targ. KRT18 v02 | AGCAGATGAAATAGCAATAGCTATCTTACCGAAAAAATCGTGTGATATTGGTGTCATC AATGACCTT |
| 328 | 67 | DNA | Human | p. seq. 8 | p. targ. KRT18 v02 | GTCTGAGTTAAGACGCTGAGAAGAGTCAATAGTAAAATCGTGTGATATTGGTGTCAT CAATGACCTT |
| 329 | 67 | DNA | Human | p. seq. 9 | p. targ. KRT18 v02 | AGCGGGCAAAGCCGGCGAACGTGGCGAGAAAGGAAAATCGTGTGATATTGGTGTCA TCAATGACCTT |
| 330 | 67 | DNA | Human | p. seq. 10 | p. targ. KRT18 v02 | AGTGTTTGATTAAAGGGATTTTAGACAGGAACGAAAATCGTGTGATATTGGTGTCAT CAATGACCTT |
| 331 | 67 | DNA | Human | p. seq. 1 | p. targ. KRT19 v01 | TATTCATAATGTTTAGACTGGATAGCGTCCAATAAAACAGACAAATTGTTGTAGTGAT CTTCCTGTC |
| 332 | 67 | DNA | Human | p. seq. 2 | p. targ. KRT19 v01 | AGGATTATCGAGCTTCAAAGCGAACCAGACCGGAAAACAGACAAATTGTTGTAGTGA TCTTCCTGTC |
| 333 | 67 | DNA | Human | p. seq. 3 | p. targ. KRT19 v01 | TGCGCAACTTCTGGTGCCGGAAACCAGGCAAAGAAAACAGACAAATTGTTGTAGTGA TCTTCCTGTC |
| 334 | 67 | DNA | Human | p. seq. 4 | p. targ. KRT19 v01 | AGGAACATTTTGCTAAACAACTTTCAACAGTTTAAAACAGACAAATTGTTGTAGTGAT CTTCCTGTC |
| 335 | 67 | DNA | Human | p. seq. 5 | p. targ. KRT19 v01 | GGATAGCCACCCTCAGAACCGCCACCCTCAGAGAAAACAGACAAATTGTTGTAGTGA TCTTCCTGTC |
| 336 | 67 | DNA | Human | p. seq. 6 | p. targ. KRT19 v01 | AAGAAAATTCATTTCAATTACCTGAGCAAAAGAAAAACAGACAAATTGTTGTAGTGAT CTTCCTGTC |
| 337 | 67 | DNA | Human | p. seq. 7 | p. targ. KRT19 v01 | AGCAGATGAAATAGCAATAGCTATCTTACCGAAAAAACAGACAAATTGTTGTAGTGAT CTTCCTGTC |
| 338 | 67 | DNA | Human | p. seq. 8 | p. targ. KRT19 v01 | GTCTGAGTTAAGACGCTGAGAAGAGTCAATAGTAAAACAGACAAATTGTTGTAGTGA TCTTCCTGTC |
| 339 | 67 | DNA | Human | p. seq. 9 | p. targ. KRT19 v01 | AGCGGGCAAAGCCGGCGAACGTGGCGAGAAAGGAAAACAGACAAATTGTTGTAGTG ATCTTCCTGTC |
| 340 | 67 | DNA | Human | p. seq. 10 | p. targ. KRT19 v01 | AGTGTTTGATTAAAGGGATTTTAGACAGGAACGAAAACAGACAAATTGTTGTAGTGA TCTTCCTGTC |
| 341 | 67 | DNA | Human | p. seq. 1 | p. targ. KRT19 v02 | TATTCATAATGTTTAGACTGGATAGCGTCCAATAAAATCTCAATGGTGGCACCAAGAA TCTTGTCCC |
| 342 | 67 | DNA | Human | p. seq. 2 | p. targ. KRT19 v02 | AGGATTATCGAGCTTCAAAGCGAACCAGACCGGAAAATCTCAATGGTGGCACCAAGA ATCTTGTCCC |
| 343 | 67 | DNA | Human | p. seq. 3 | p. targ. KRT19 v02 | TGCGCAACTTCTGGTGCCGGAAACCAGGCAAAGAAAATCTCAATGGTGGCACCAAG AATCTTGTCCC |
| 344 | 67 | DNA | Human | p. seq. 4 | p. targ. KRT19 v02 | AGGAACATTTTGCTAAACAACTTTCAACAGTTTAAAATCTCAATGGTGGCACCAAGAA TCTTGTCCC |
| 345 | 67 | DNA | Human | p. seq. 5 | p. targ. KRT19 v02 | GGATAGCCACCCTCAGAACCGCCACCCTCAGAGAAAATCTCAATGGTGGCACCAAG AATCTTGTCCC |
| 346 | 67 | DNA | Human | p. seq. 6 | p. targ. KRT19 v02 | AAGAAAATTCATTTCAATTACCTGAGCAAAAGAAAAATCTCAATGGTGGCACCAAGAA TCTTGTCCC |
| 347 | 67 | DNA | Human | p. seq. 7 | p. targ. KRT19 v02 | AGCAGATGAAATAGCAATAGCTATCTTACCGAAAAAATCTCAATGGTGGCACCAAGA ATCTTGTCCC |
| 348 | 67 | DNA | Human | p. seq. 8 | p. targ. KRT19 v02 | GTCTGAGTTAAGACGCTGAGAAGAGTCAATAGTAAAATCTCAATGGTGGCACCAAGA ATCTTGTCCC |
| 349 | 67 | DNA | Human | p. seq. 9 | p. targ. KRT19 v02 | AGCGGGCAAAGCCGGCGAACGTGGCGAGAAAGGAAAATCTCAATGGTGGCACCAA GAATCTTGTCCC |
| 350 | 67 | DNA | Human | p. seq. 10 | p. targ. KRT19 v02 | AGTGTTTGATTAAAGGGATTTTAGACAGGAACGAAAATCTCAATGGTGGCACCAAGA ATCTTGTCCC |
| 351 | 67 | DNA | Human | p. seq. 1 | p. targ. NR3C3 v01 | TATTCATAATGTTTAGACTGGATAGCGTCCAATAAAAAGGTAATGACTCGAAGCTGTA TTGTGGGCT |
| 352 | 67 | DNA | Human | p. seq. 2 | p. targ. NR3C3 v01 | AGGATTATCGAGCTTCAAAGCGAACCAGACCGGAAAAAGGTAATGACTCGAAGCTGT ATTGTGGGCT |
| 353 | 67 | DNA | Human | p. seq. 3 | p. targ. NR3C3 v01 | TGCGCAACTTCTGGTGCCGGAAACCAGGCAAAGAAAAAGGTAATGACTCGAAGCTG TATTGTGGGCT |
| 354 | 67 | DNA | Human | p. seq. 4 | p. targ. NR3C3 v01 | AGGAACATTTTGCTAAACAACTTTCAACAGTTTAAAAAGGTAATGACTCGAAGCTGTA TTGTGGGCT |
| 355 | 67 | DNA | Human | p. seq. 5 | p. targ. NR3C3 v01 | GGATAGCCACCCTCAGAACCGCCACCCTCAGAGAAAAAGGTAATGACTCGAAGCTG TATTGTGGGCT |
| 356 | 67 | DNA | Human | p. seq. 6 | p. targ. NR3C3 v01 | AAGAAAATTCATTTCAATTACCTGAGCAAAAGAAAAAAGGTAATGACTCGAAGCTGTA TTGTGGGCT |
| 357 | 67 | DNA | Human | p. seq. 7 | p. targ. NR3C3 v01 | AGCAGATGAAATAGCAATAGCTATCTTACCGAAAAAAAGGTAATGACTCGAAGCTGT ATTGTGGGCT |
| 358 | 67 | DNA | Human | p. seq. 8 | p. targ. NR3C3 v01 | GTCTGAGTTAAGACGCTGAGAAGAGTCAATAGTAAAAAGGTAATGACTCGAAGCTGT ATTGTGGGCT |
| 359 | 67 | DNA | Human | p. seq. 9 | p. targ. NR3C3 v01 | AGCGGGCAAAGCCGGCGAACGTGGCGAGAAAGGAAAAAGGTAATGACTCGAAGCT GTATTGTGGGCT |
| 360 | 67 | DNA | Human | p. seq. 10 | p. targ. NR3C3 v01 | AGTGTTTGATTAAAGGGATTTTAGACAGGAACGAAAAAGGTAATGACTCGAAGCTGT ATTGTGGCT |
| 361 | 67 | DNA | Human | p. seq. 1 | p. targ. NR3C3 v02 | TATTCATAATGTTTAGACTGGATAGCGTCCAATAAAAGGCAGGATAGGCACGTGGAT GAAATCCATC |
| 362 | 67 | DNA | Human | p. seq. 2 | p. targ. NR3C3 v02 | AGGATTATCGAGCTTCAAAGCGAACCAGACCGGAAAAGGCAGGATAGGCACGTGGA TGAAATCCATC |
| 363 | 67 | DNA | Human | p. seq. 3 | p. targ. NR3C3 v02 | TGCGCAACTTCTGGTGCCGGAAACCAGGCAAAGAAAAGGCAGGATAGGCACGTGGA TGAAATCCATC |
| 364 | 67 | DNA | Human | p. seq. 4 | p. targ. NR3C3 v02 | AGGAACATTTTGCTAAACAACTTTCAACAGTTTAAAAGGCAGGATAGGCACGTGGAT GAAATCCATC |
| 365 | 67 | DNA | Human | p. seq. 5 | p. targ. NR3C3 v02 | GGATAGCCACCCTCAGAACCGCCACCCTCAGAGAAAAGGCAGGATAGGCACGTGGA TGAAATCCATC |
| 366 | 67 | DNA | Human | p. seq. 6 | p. targ. NR3C3 v02 | AAGAAAATTCATTTCAATTACCTGAGCAAAAGAAAAAGGCAGGATAGGCACGTGGAT GAAATCCATC |
| 367 | 67 | DNA | Human | p. seq. 7 | p. targ. NR3C3 v02 | AGCAGATGAAATAGCAATAGCTATCTTACCGAAAAAAGGCAGGATAGGCACGTGGAT GAAATCCATC |
| 368 | 67 | DNA | Human | p. seq. 8 | p. targ. NR3C3 v02 | GTCTGAGTTAAGACGCTGAGAAGAGTCAATAGTAAAAGGCAGGATAGGCACGTGGA TGAAATCCATC |
| 369 | 67 | DNA | Human | p. seq. 9 | p. targ. NR3C3 v02 | AGCGGGCAAAGCCGGCGAACGTGGCGAGAAAGGAAAAGGCAGGATAGGCACGTGG ATGAAATCCATC |
| 370 | 67 | DNA | Human | p. seq. 10 | p. targ. NR3C3 v02 | AGTGTTTGATTAAAGGGATTTTAGACAGGAACGAAAAGGCAGGATAGGCACGTGGA TGAAATCCATC |
| 371 | 67 | DNA | Human | p. seq. 1 | p. targ. GATA6 v01 | TATTCATAATGTTTAGACTGGATAGCGTCCAATAAAAAGGGATGCGAAGCGTAGGAA CTGAGCAGCA |
| 372 | 67 | DNA | Human | p. seq. 2 | p. targ. GATA6 v01 | AGGATTATCGAGCTTCAAAGCGAACCAGACCGGAAAAAGGGATGCGAAGCGTAGGA ACTGAGCAGCA |
| 373 | 67 | DNA | Human | p. seq. 3 | p. targ. GATA6 v01 | TGCGCAACTTCTGGTGCCGGAAACCAGGCAAAGAAAAAGGGATGCGAAGCGTAGGA ACTGAGCAGCA |
| 374 | 67 | DNA | Human | p. seq. 4 | p. targ. GATA6 v01 | AGGAACATTTTGCTAAACAACTTTCAACAGTTTAAAAAGGGATGCGAAGCGTAGGAA CTGAGCAGCA |
| 375 | 67 | DNA | Human | p. seq. 5 | p. targ. GATA6 v01 | GGATAGCCACCCTCAGAACCGCCACCCTCAGAGAAAAAGGGATGCGAAGCGTAGGA ACTGAGCAGCA |
| 376 | 67 | DNA | Human | p. seq. 6 | p. targ. GATA6 v01 | AAGAAAATTCATTTCAATTACCTGAGCAAAAGAAAAAAGGGATGCGAAGCGTAGGAA CTGAGCAGCA |
| 377 | 67 | DNA | Human | p. seq. 7 | p. targ. GATA6 v01 | AGCAGATGAAATAGCAATAGCTATCTTACCGAAAAAAAGGGATGCGAAGCGTAGGA ACTGAGCAGCA |
| 378 | 67 | DNA | Human | p. seq. 8 | p. targ. GATA6 v01 | GTCTGAGTTAAGACGCTGAGAAGAGTCAATAGTAAAAAGGGATGCGAAGCGTAGGA ACTGAGCAGCA |
| 379 | 67 | DNA | Human | p. seq. 9 | p. targ. GATA6 v01 | AGCGGGCAAAGCCGGCGAACGTGGCGAGAAAGGAAAAAGGGATGCGAAGCGTAGG AACTGAGCAGCA |
| 380 | 67 | DNA | Human | p. seq. 10 | p. targ. GATA6 v01 | AGTGTTTGATTAAAGGGATTTTAGACAGGAACGAAAAAGGGATGCGAAGCGTAGGA ACTGAGCAGCA |
| 381 | 67 | DNA | Human | p. seq. 1 | p. targ. GATA6 v02 | TATTCATAATGTTTAGACTGGATAGCGTCCAATAAAACGAGGTCAGTGAACAGCAGC AAGTCCTCCC |
| 382 | 67 | DNA | Human | p. seq. 2 | p. targ. GATA6 v02 | AGGATTATCGAGCTTCAAAGCGAACCAGACCGGAAAACGAGGTCAGTGAACAGCAG CAAGTCCTCCC |
| 383 | 67 | DNA | Human | p. seq. 3 | p. targ. GATA6 v02 | TGCGCAACTTCTGGTGCCGGAAACCAGGCAAAGAAAACGAGGTCAGTGAACAGCAG CAAGTCCTCCC |
| 384 | 67 | DNA | Human | p. seq. 4 | p. targ. GATA6 v02 | AGGAACATTTTGCTAAACAACTTTCAACAGTTTAAAACGAGGTCAGTGAACAGCAGC AAGTCCTCCC |
| 385 | 67 | DNA | Human | p. seq. 5 | p. targ. GATA6 v02 | GGATAGCCACCCTCAGAACCGCCACCCTCAGAGAAAACGAGGTCAGTGAACAGCAG CAAGTCCTCCC |
| 386 | 67 | DNA | Human | p. seq. 6 | p. targ. GATA6 v02 | AAGAAAATTCATTTCAATTACCTGAGCAAAAGAAAAACGAGGTCAGTGAACAGCAGC AAGTCCTCCC |
| 387 | 67 | DNA | Human | p. seq. 7 | p. targ. GATA6 v02 | AGCAGATGAAATAGCAATAGCTATCTTACCGAAAAAACGAGGTCAGTGAACAGCAGC AAGTCCTCCC |
| 388 | 67 | DNA | Human | p. seq. 8 | p. targ. GATA6 v02 | GTCTGAGTTAAGACGCTGAGAAGAGTCAATAGTAAAACGAGGTCAGTGAACAGCAG CAAGTCCTCCC |
| 389 | 67 | DNA | Human | p. seq. 9 | p. targ. GATA6 v02 | AGCGGGCAAAGCCGGCGAACGTGGCGAGAAAGGAAAACGAGGTCAGTGAACAGCA GCAAGTCCTCCC |
| 390 | 67 | DNA | Human | p. seq. 10 | p. targ. GATA6 v02 | AGTGTTTGATTAAAGGGATTTTAGACAGGAACGAAAACGAGGTCAGTGAACAGCAGC AAGTCCTCCC |
| 391 | 67 | DNA | Human | p. seq. 1 | p. targ. LPAR5 v01 | TATTCATAATGTTTAGACTGGATAGCGTCCAATAAAAGCAGTGCGTAGTAGGAGAGA CGAACGGGCA |
| 392 | 67 | DNA | Human | p. seq. 2 | p. targ. LPAR5 v01 | AGGATTATCGAGCTTCAAAGCGAACCAGACCGGAAAAGCAGTGCGTAGTAGGAGAG ACGAACGGGCA |
| 393 | 67 | DNA | Human | p. seq. 3 | p. targ. LPAR5 v01 | TGCGCAACTTCTGGTGCCGGAAACCAGGCAAAGAAAAGCAGTGCGTAGTAGGAGAG ACGAACGGGCA |
| 394 | 67 | DNA | Human | p. seq. 4 | p. targ. LPAR5 v01 | AGGAACATTTTGCTAAACAACTTTCAACAGTTTAAAAGCAGTGCGTAGTAGGAGAGA CGAACGGGCA |
| 395 | 67 | DNA | Human | p. seq. 5 | p. targ. LPAR5 v01 | GGATAGCCACCCTCAGAACCGCCACCCTCAGAGAAAAGCAGTGCGTAGTAGGAGAG ACGAACGGGCA |
| 396 | 67 | DNA | Human | p. seq. 6 | p. targ. LPAR5 v01 | AAGAAAATTCATTTCAATTACCTGAGCAAAAGAAAAAGCAGTGCGTAGTAGGAGAGA CGAACGGGCA |
| 397 | 67 | DNA | Human | p. seq. 7 | p. targ. LPAR5 v01 | AGCAGATGAAATAGCAATAGCTATCTTACCGAAAAAAGCAGTGCGTAGTAGGAGAGA CGAACGGGCA |
| 398 | 67 | DNA | Human | p. seq. 8 | p. targ. LPAR5 v01 | GTCTGAGTTAAGACGCTGAGAAGAGTCAATAGTAAAAGCAGTGCGTAGTAGGAGAG ACGAACGGGCA |
| 399 | 67 | DNA | Human | p. seq. 9 | p. targ. LPAR5 v01 | AGCGGGCAAAGCCGGCGAACGTGGCGAGAAAGGAAAAGCAGTGCGTAGTAGGAGA GACGAACGGGCA |
| 400 | 67 | DNA | Human | p. seq. 10 | p. targ. LPAR5 v01 | AGTGTTTGATTAAAGGGATTTTAGACAGGAACGAAAAGCAGTGCGTAGTAGGAGAG ACGAACGGGCA |
| 401 | 67 | DNA | Human | p. seq. 1 | p. targ. LPAR5 v02 | TATTCATAATGTTTAGACTGGATAGCGTCCAATAAAACACTGTGTGAAGGAAGACAG AGAGTGGGAG |
| 402 | 67 | DNA | Human | p. seq. 2 | p. targ. LPAR5 v02 | AGGATTATCGAGCTTCAAAGCGAACCAGACCGGAAAACACTGTGTGAAGGAAGACA GAGAGTGGGAG |
| 403 | 67 | DNA | Human | p. seq. 3 | p. targ. LPAR5 v02 | TGCGCAACTTCTGGTGCCGGAAACCAGGCAAAGAAAACACTGTGTGAAGGAAGACA GAGAGTGGGAG |
| 404 | 67 | DNA | Human | p. seq. 4 | p. targ. LPAR5 v02 | AGGAACATTTTGCTAAACAACTTTCAACAGTTTAAAACACTGTGTGAAGGAAGACAG AGAGTGGGAG |
| 405 | 67 | DNA | Human | p. seq. 5 | p. targ. LPAR5 v02 | GGATAGCCACCCTCAGAACCGCCACCCTCAGAGAAAACACTGTGTGAAGGAAGACA GAGAGTGGGAG |
| 406 | 67 | DNA | Human | p. seq. 6 | p. targ. LPAR5 v02 | AAGAAAATTCATTTCAATTACCTGAGCAAAAGAAAAACACTGTGTGAAGGAAGACAG AGAGTGGGAG |
| 407 | 67 | DNA | Human | p. seq. 7 | p. targ. LPAR5 v02 | AGCAGATGAAATAGCAATAGCTATCTTACCGAAAAAACACTGTGTGAAGGAAGACAG AGAGTGGGAG |
| 408 | 67 | DNA | Human | p. seq. 8 | p. targ. LPAR5 v02 | GTCTGAGTTAAGACGCTGAGAAGAGTCAATAGTAAAACACTGTGTGAAGGAAGACA GAGAGTGGGAG |
| 409 | 67 | DNA | Human | p. seq. 9 | p. targ. LPAR5 v02 | AGCGGGCAAAGCCGGCGAACGTGGCGAGAAAGGAAAACACTGTGTGAAGGAAGAC AGAGAGTGGGAG |
| 410 | 67 | DNA | Human | p. seq. 10 | p. targ. LPAR5 v02 | AGTGTTTGATTAAAGGGATTTTAGACAGGAACGAAAACACTGTGTGAAGGAAGACAG AGAGTGGGAG |
| 411 | 67 | DNA | Human | p. seq. 1 | p. targ. ERBB2 v01 | TATTCATAATGTTTAGACTGGATAGCGTCCAATAAAAAGGGCATAGTTGTCCTCAAAG AGCTGGGTG |
| 412 | 67 | DNA | Human | p. seq. 2 | p. targ. ERBB2 v01 | AGGATTATCGAGCTTCAAAGCGAACCAGACCGGAAAAAGGGCATAGTTGTCCTCAAA GAGCTGGGTG |
| 413 | 67 | DNA | Human | p. seq. 3 | p. targ. ERBB2 v01 | TGCGCAACTTCTGGTGCCGGAAACCAGGCAAAGAAAAAGGGCATAGTTGTCCTCAA AGAGCTGGGTG |
| 414 | 67 | DNA | Human | p. seq. 4 | p. targ. ERBB2 v01 | AGGAACATTTTGCTAAACAACTTTCAACAGTTTAAAAAGGGCATAGTTGTCCTCAAAG AGCTGGGTG |
| 415 | 67 | DNA | Human | p. seq. 5 | p. targ. ERBB2 v01 | GGATAGCCACCCTCAGAACCGCCACCCTCAGAGAAAAAGGGCATAGTTGTCCTCAAA GAGCTGGGTG |
| 416 | 67 | DNA | Human | p. seq. 6 | p. targ. ERBB2 v01 | AAGAAAATTCATTTCAATTACCTGAGCAAAAGAAAAAAGGGCATAGTTGTCCTCAAAG AGCTGGGTG |
| 417 | 67 | DNA | Human | p. seq. 7 | p. targ. ERBB2 v01 | AGCAGATGAAATAGCAATAGCTATCTTACCGAAAAAAAGGGCATAGTTGTCCTCAAA GAGCTGGGTG |
| 418 | 67 | DNA | Human | p. seq. 8 | p. targ. ERBB2 v01 | GTCTGAGTTAAGACGCTGAGAAGAGTCAATAGTAAAAAGGGCATAGTTGTCCTCAAA GAGCTGGGTG |
| 419 | 67 | DNA | Human | p. seq. 9 | p. targ. ERBB2 v01 | AGCGGGCAAAGCCGGCGAACGTGGCGAGAAAGGAAAAAGGGCATAGTTGTCCTCA AAGAGCTGGGTG |
| 420 | 67 | DNA | Human | p. seq. 10 | p. targ. ERBB2 v01 | AGTGTTTGATTAAAGGGATTTTAGACAGGAACGAAAAAGGGCATAGTTGTCCTCAAA GAGCTGGGTG |
| 421 | 67 | DNA | Human | p. seq. 1 | p. targ. ERBB2 v02 | TATTCATAATGTTTAGACTGGATAGCGTCCAATAAAACCACAACAGTACATGTCACTC TCATCACTG |
| 422 | 67 | DNA | Human | p. seq. 2 | p. targ. ERBB2 v02 | AGGATTATCGAGCTTCAAAGCGAACCAGACCGGAAAACCACAACAGTACATGTCACT CTCATCACTG |
| 423 | 67 | DNA | Human | p. seq. 3 | p. targ. ERBB2 v02 | TGCGCAACTTCTGGTGCCGGAAACCAGGCAAAGAAAACCACAACAGTACATGTCACT CTCATCACTG |
| 424 | 67 | DNA | Human | p. seq. 4 | p. targ. ERBB2 v02 | AGGAACATTTTGCTAAACAACTTTCAACAGTTTAAAACCACAACAGTACATGTCACTC TCATCACTG |
| 425 | 67 | DNA | Human | p. seq. 5 | p. targ. ERBB2 v02 | GGATAGCCACCCTCAGAACCGCCACCCTCAGAGAAAACCACAACAGTACATGTCACT CTCATCACTG |
| 426 | 67 | DNA | Human | p. seq. 6 | p. targ. ERBB2 v02 | AAGAAAATTCATTTCAATTACCTGAGCAAAAGAAAAACCACAACAGTACATGTCACTC TCATCACTG |
| 427 | 67 | DNA | Human | p. seq. 7 | p. targ. ERBB2 v02 | AGCAGATGAAATAGCAATAGCTATCTTACCGAAAAAACCACAACAGTACATGTCACT CTCATCACTG |
| 428 | 67 | DNA | Human | p. seq. 8 | p. targ. ERBB2 v02 | GTCTGAGTTAAGACGCTGAGAAGAGTCAATAGTAAAACCACAACAGTACATGTCACT CTCATCACTG |
| 429 | 67 | DNA | Human | p. seq. 9 | p. targ. ERBB2 v02 | AGCGGGCAAAGCCGGCGAACGTGGCGAGAAAGGAAAACCACAACAGTACATGTCAC TCTCATCACTG |
| 430 | 67 | DNA | Human | p. seq. 10 | p. targ. ERBB2 v02 | AGTGTTTGATTAAAGGGATTTTAGACAGGAACGAAAACCACAACAGTACATGTCACT CTCATCACTG |
| 431 | 67 | DNA | Human | p. seq. 1 | p. targ. TP53 v01 | TATTCATAATGTTTAGACTGGATAGCGTCCAATAAAAAGTTCTAGACCCCATGTAATA AAAGGTGGT |
| 432 | 67 | DNA | Human | p. seq. 2 | p. targ. TP53 v01 | AGGATTATCGAGCTTCAAAGCGAACCAGACCGGAAAAAGTTCTAGACCCCATGTAAT AAAAGGTGGT |
| 433 | 67 | DNA | Human | p. seq. 3 | p. targ. TP53 v01 | TGCGCAACTTCTGGTGCCGGAAACCAGGCAAAGAAAAAGTTCTAGACCCCATGTAAT AAAAGGTGGT |
| 434 | 67 | DNA | Human | p. seq. 4 | p. targ. TP53 v01 | AGGAACATTTTGCTAAACAACTTTCAACAGTTTAAAAAGTTCTAGACCCCATGTAATA AAAGGTGGT |
| 435 | 67 | DNA | Human | p. seq. 5 | p. targ. TP53 v01 | GGATAGCCACCCTCAGAACCGCCACCCTCAGAGAAAAAGTTCTAGACCCCATGTAAT AAAAGGTGGT |
| 436 | 67 | DNA | Human | p. seq. 6 | p. targ. TP53 v01 | AAGAAAATTCATTTCAATTACCTGAGCAAAAGAAAAAAGTTCTAGACCCCATGTAATA AAAGGTGGT |
| 437 | 67 | DNA | Human | p. seq. 7 | p. targ. TP53 v01 | AGCAGATGAAATAGCAATAGCTATCTTACCGAAAAAAAGTTCTAGACCCCATGTAATA AAAGGTGGT |
| 438 | 67 | DNA | Human | p. seq. 8 | p. targ. TP53 v01 | GTCTGAGTTAAGACGCTGAGAAGAGTCAATAGTAAAAAGTTCTAGACCCCATGTAAT AAAAGGTGGT |
| 439 | 67 | DNA | Human | p. seq. 9 | p. targ. TP53 v01 | AGCGGGCAAAGCCGGCGAACGTGGCGAGAAAGGAAAAAGTTCTAGACCCCATGTAA TAAAAGGTGGT |
| 440 | 67 | DNA | Human | p. seq. 10 | p. targ. TP53 v01 | AGTGTTTGATTAAAGGGATTTTAGACAGGAACGAAAAAGTTCTAGACCCCATGTAAT AAAAGGTGGT |
| 441 | 67 | DNA | Human | p. seq. 1 | p. targ. PDX1 v01 | TATTCATAATGTTTAGACTGGATAGCGTCCAATAAAATTCCAATGTGTATGGTACAGT TTCCATTGT |
| 442 | 67 | DNA | Human | p. seq. 2 | p. targ. PDX1 v01 | AGGATTATCGAGCTTCAAAGCGAACCAGACCGGAAAATTCCAATGTGTATGGTACAG TTTCCATTGT |
| 443 | 67 | DNA | Human | p. seq. 3 | p. targ. PDX1 v01 | TGCGCAACTTCTGGTGCCGGAAACCAGGCAAAGAAAATTCCAATGTGTATGGTACAG TTTCCATTGT |
| 444 | 67 | DNA | Human | p. seq. 4 | p. targ. PDX1 v01 | AGGAACATTTTGCTAAACAACTTTCAACAGTTTAAAATTCCAATGTGTATGGTACAGT TTCCATTGT |
| 445 | 67 | DNA | Human | p. seq. 5 | p. targ. PDX1 v01 | GGATAGCCACCCTCAGAACCGCCACCCTCAGAGAAAATTCCAATGTGTATGGTACAG TTTCCATTGT |
| 446 | 67 | DNA | Human | p. seq. 6 | p. targ. PDX1 v01 | AAGAAAATTCATTTCAATTACCTGAGCAAAAGAAAAATTCCAATGTGTATGGTACAGT TTCCATTGT |
| 447 | 67 | DNA | Human | p. seq. 7 | p. targ. PDX1 v01 | AGCAGATGAAATAGCAATAGCTATCTTACCGAAAAAATTCCAATGTGTATGGTACAGT TTCCATTGT |
| 448 | 67 | DNA | Human | p. seq. 8 | p. targ. PDX1 v01 | GTCTGAGTTAAGACGCTGAGAAGAGTCAATAGTAAAATTCCAATGTGTATGGTACAG TTTCCATTGT |
| 449 | 67 | DNA | Human | p. seq. 9 | p. targ. PDX1 v01 | AGCGGGCAAAGCCGGCGAACGTGGCGAGAAAGGAAAATTCCAATGTGTATGGTACA GTTTCCATTGT |
| 450 | 67 | DNA | Human | p. seq. 10 | p. targ. PDX1 v01 | AGTGTTTGATTAAAGGGATTTTAGACAGGAACGAAAATTCCAATGTGTATGGTACAG TTTCCATTGT |
| 451 | 67 | DNA | Human | p. seq. 1 | p. targ. PDX1 v02 | TATTCATAATGTTTAGACTGGATAGCGTCCAATAAAAGTCTTTGAAGAGCCGGCTTCT CTAAACAGG |
| 452 | 67 | DNA | Human | p. seq. 2 | p. targ. PDX1 v02 | AGGATTATCGAGCTTCAAAGCGAACCAGACCGGAAAAGTCTTTGAAGAGCCGGCTTC TCTAAACAGG |
| 453 | 67 | DNA | Human | p. seq. 3 | p. targ. PDX1 v02 | TGCGCAACTTCTGGTGCCGGAAACCAGGCAAAGAAAAGTCTTTGAAGAGCCGGCTT CTCTAAACAGG |
| 454 | 67 | DNA | Human | p. seq. 4 | p. targ. PDX1 v02 | AGGAACATTTTGCTAAACAACTTTCAACAGTTTAAAAGTCTTTGAAGAGCCGGCTTCT CTAAACAGG |
| 455 | 67 | DNA | Human | p. seq. 5 | p. targ. PDX1 v02 | GGATAGCCACCCTCAGAACCGCCACCCTCAGAGAAAAGTCTTTGAAGAGCCGGCTTC TCTAAACAGG |
| 456 | 67 | DNA | Human | p. seq. 6 | p. targ. PDX1 v02 | AAGAAAATTCATTTCAATTACCTGAGCAAAAGAAAAAGTCTTTGAAGAGCCGGCTTCT CTAAACAGG |
| 457 | 67 | DNA | Human | p. seq. 7 | p. targ. PDX1 v02 | AGCAGATGAAATAGCAATAGCTATCTTACCGAAAAAAGTCTTTGAAGAGCCGGCTTC TCTAAACAGG |
| 458 | 67 | DNA | Human | p. seq. 8 | p. targ. PDX1 v02 | GTCTGAGTTAAGACGCTGAGAAGAGTCAATAGTAAAAGTCTTTGAAGAGCCGGCTTC TCTAAACAGG |
| 459 | 67 | DNA | Human | p. seq. 9 | p. targ. PDX1 v02 | AGCGGGCAAAGCCGGCGAACGTGGCGAGAAAGGAAAAGTCTTTGAAGAGCCGGCT TCTCTAAACAGG |
| 460 | 67 | DNA | Human | p. seq. 10 | p. targ. PDX1 v02 | AGTGTTTGATTAAAGGGATTTTAGACAGGAACGAAAAGTCTTTGAAGAGCCGGCTTC TCTAAACAGG |
| 461 | 67 | DNA | Human | p. seq. 1 | p. targ. PDX1 v03 | TATTCATAATGTTTAGACTGGATAGCGTCCAATAAAAAGCCACAAACAACGCCAATCC AGTATCACT |
| 462 | 67 | DNA | Human | p. seq. 2 | p. targ. PDX1 v03 | AGGATTATCGAGCTTCAAAGCGAACCAGACCGGAAAAAGCCACAAACAACGCCAATC CAGTATCACT |
| 463 | 67 | DNA | Human | p. seq. 3 | p. targ. PDX1 v03 | TGCGCAACTTCTGGTGCCGGAAACCAGGCAAAGAAAAAGCCACAAACAACGCCAAT CCAGTATCACT |
| 464 | 67 | DNA | Human | p. seq. 4 | p. targ. PDX1 v03 | AGGAACATTTTGCTAAACAACTTTCAACAGTTTAAAAAGCCACAAACAACGCCAATCC AGTATCACT |
| 465 | 67 | DNA | Human | p. seq. 5 | p. targ. PDX1 v03 | GGATAGCCACCCTCAGAACCGCCACCCTCAGAGAAAAAGCCACAAACAACGCCAATC CAGTATCACT |
| 466 | 67 | DNA | Human | p. seq. 6 | p. targ. PDX1 v03 | AAGAAAATTCATTTCAATTACCTGAGCAAAAGAAAAAAGCCACAAACAACGCCAATCC AGTATCACT |
| 467 | 67 | DNA | Human | p. seq. 7 | p. targ. PDX1 v03 | AGCAGATGAAATAGCAATAGCTATCTTACCGAAAAAAAGCCACAAACAACGCCAATC CAGTATCACT |
| 468 | 67 | DNA | Human | p. seq. 8 | p. targ. PDX1 v03 | GTCTGAGTTAAGACGCTGAGAAGAGTCAATAGTAAAAAGCCACAAACAACGCCAATC CAGTATCACT |
| 469 | 67 | DNA | Human | p. seq. 9 | p. targ. PDX1 v03 | AGCGGGCAAAGCCGGCGAACGTGGCGAGAAAGGAAAAAGCCACAAACAACGCCAA TCCAGTATCACT |
| 470 | 67 | DNA | Human | p. seq. 10 | p. targ. PDX1 v03 | AGTGTTTGATTAAAGGGATTTTAGACAGGAACGAAAAAGCCACAAACAACGCCAATC CAGTATCACT |
| 471 | 67 | DNA | Human | p. seq. 1 | p. targ. PDX1 v04 | TATTCATAATGTTTAGACTGGATAGCGTCCAATAAAATCACTAAAAGAGGGCCCCAAT ACTACAAAA |
| 472 | 67 | DNA | Human | p. seq. 2 | p. targ. PDX1 v04 | AGGATTATCGAGCTTCAAAGCGAACCAGACCGGAAAATCACTAAAAGAGGGCCCCA ATACTACAAAA |
| 473 | 67 | DNA | Human | p. seq. 3 | p. targ. PDX1 v04 | TGCGCAACTTCTGGTGCCGGAAACCAGGCAAAGAAAATCACTAAAAGAGGGCCCCA ATACTACAAAA |
| 474 | 67 | DNA | Human | p. seq. 4 | p. targ. PDX1 v04 | AGGAACATTTTGCTAAACAACTTTCAACAGTTTAAAATCACTAAAAGAGGGCCCCAAT ACTACAAAA |
| 475 | 67 | DNA | Human | p. seq. 5 | p. targ. PDX1 v04 | GGATAGCCACCCTCAGAACCGCCACCCTCAGAGAAAATCACTAAAAGAGGGCCCCA ATACTACAAAA |
| 476 | 67 | DNA | Human | p. seq. 6 | p. targ. PDX1 v04 | AAGAAAATTCATTTCAATTACCTGAGCAAAAGAAAAATCACTAAAAGAGGGCCCCAAT ACTACAAAA |
| 477 | 67 | DNA | Human | p. seq. 7 | p. targ. PDX1 v04 | AGCAGATGAAATAGCAATAGCTATCTTACCGAAAAAATCACTAAAAGAGGGCCCCAA TACTACAAAA |
| 478 | 67 | DNA | Human | p. seq. 8 | p. targ. PDX1 v04 | GTCTGAGTTAAGACGCTGAGAAGAGTCAATAGTAAAATCACTAAAAGAGGGCCCCAA TACTACAAAA |
| 479 | 67 | DNA | Human | p. seq. 9 | p. targ. PDX1 v04 | AGCGGGCAAAGCCGGCGAACGTGGCGAGAAAGGAAAATCACTAAAAGAGGGCCCC AATACTACAAAA |
| 480 | 67 | DNA | Human | p. seq. 10 | p. targ. PDX1 v04 | AGTGTTTGATTAAAGGGATTTTAGACAGGAACGAAAATCACTAAAAGAGGGCCCCAA TACTACAAAA |
| 481 | 67 | DNA | Human | p. seq. 1 | p. targ. PDX1 v05 | TATTCATAATGTTTAGACTGGATAGCGTCCAATAAAAACAAAAACCAATGGCTCTTTT TCTTCCGGA |
| 482 | 67 | DNA | Human | p. seq. 2 | p. targ. PDX1 v05 | AGGATTATCGAGCTTCAAAGCGAACCAGACCGGAAAAACAAAAACCAATGGCTCTTT TTCTTCCGGA |
| 483 | 67 | DNA | Human | p. seq. 3 | p. targ. PDX1 v05 | TGCGCAACTTCTGGTGCCGGAAACCAGGCAAAGAAAAACAAAAACCAATGGCTCTTT TTCTTCCGGA |
| 484 | 67 | DNA | Human | p. seq. 4 | p. targ. PDX1 v05 | AGGAACATTTTGCTAAACAACTTTCAACAGTTTAAAAACAAAAACCAATGGCTCTTTTT CTTCCGGA |
| 485 | 67 | DNA | Human | p. seq. 5 | p. targ. PDX1 v05 | GGATAGCCACCCTCAGAACCGCCACCCTCAGAGAAAAACAAAAACCAATGGCTCTTT TTCTTCCGGA |
| 486 | 67 | DNA | Human | p. seq. 6 | p. targ. PDX1 v05 | AAGAAAATTCATTTCAATTACCTGAGCAAAAGAAAAAACAAAAACCAATGGCTCTTTT TCTTCCGGA |
| 487 | 67 | DNA | Human | p. seq. 7 | p. targ. PDX1 v05 | AGCAGATGAAATAGCAATAGCTATCTTACCGAAAAAAACAAAAACCAATGGCTCTTTT TCTTCCGGA |
| 488 | 67 | DNA | Human | p. seq. 8 | p. targ. PDX1 v05 | GTCTGAGTTAAGACGCTGAGAAGAGTCAATAGTAAAAACAAAAACCAATGGCTCTTT TTCTTCCGGA |
| 489 | 67 | DNA | Human | p. seq. 9 | p. targ. PDX1 v05 | AGCGGGCAAAGCCGGCGAACGTGGCGAGAAAGGAAAAACAAAAACCAATGGCTCTT TTTCTTCCGGA |
| 490 | 67 | DNA | Human | p. seq. 10 | p. targ. PDX1 v05 | AGTGTTTGATTAAAGGGATTTTAGACAGGAACGAAAAACAAAAACCAATGGCTCTTTT TCTTCCGGA |

While in the above, a preferred embodiment has been described with reference to the accompanying drawings, the skilled person will understand that this embodiment was provided for illustrative purpose only and should by no means be construed to limit the scope of the present invention, which is defined by the claims.

Whenever a relative term, such as "about", "substantially" or "approximately" is used in this specification, such a term should also be construed to also include the exact term. That is, e.g., "substantially straight" should be construed to also include "(exactly) straight".

Whenever steps were recited in the above or also in the appended claims, it should be noted that the order in which the steps are recited in this text may be accidental. That is, unless otherwise specified or unless clear to the skilled person, the order in which steps are recited may be accidental. That is, when the present document states, e.g., that a method comprises steps (A) and (B), this does not necessarily mean that step (A) precedes step (B), but it is also possible that step (A) is performed (at least partly) simultaneously with step (B) or that step (B) precedes step (A). Furthermore, when a step (X) is said to precede another step (Z), this does not imply that there is no step between steps (X) and (Z). That is, step (X) preceding step (Z) encompasses the situation that step (X) is performed directly before step (Z), but also the situation that (X) is performed before one or more steps (Y1), ..., followed by step (Z). Corresponding considerations apply when terms like "after" or "before" are used.

## Claims

1. A probe for RNA fluorescence in situ hybridization (FISH) comprising
a marker molecule,
a targeting molecule comprising 22 to 55 nt comprising a spacer sequence, and
a target binding sequence capable of binding to a target molecule,
wherein the marker molecule is connected to the targeting molecule via the spacer sequence.

2. The probe according to the preceding claim, wherein the probe comprises a marker molecule binding sequence comprising 20 to 40 nt, wherein the probe comprises an oligonucleotide molecule comprising the marker molecule binding sequence, the spacer sequence, and the target binding sequence.

3. The probe according to any of the preceding claims, wherein the marker molecule comprises a DNA-nanostructure, wherein the DNA-nanostructure comprises at least one of: a 2D DNA-nanostructure, and a 3D DNA-nanostructure.

4. The probe according to any of the preceding claims, wherein the probe comprises a DNA oligonucleotide comprising a length between 57 and 92 nt, wherein the DNA oligonucleotide comprises the target binding sequence, wherein 20 to 55 nt of the DNA oligonucleotide comprises the target binding sequence.

5. The probe according to any of the preceding claims, wherein
the targeting molecule comprises a nucleotide sequence complementary to an RNA target sequence of a RNA to be detected,
the marker molecule comprising at least one fluorophore comprises at least 10 fluorophore molecules, preferably at least 30 molecules, more preferably at least 40 fluorophore molecules incorporated in its structure, wherein the marker molecule comprising the at least one fluorophore comprises at most 80 fluorophore molecules, preferably at most 70 fluorophore molecules incorporated in its structure.

6. The probe according to any of the preceding claims and with the features of claim 4, wherein the DNA-nanostructure comprises a DNA design comprising a sequence of staples, wherein the sequence of staples is configured to bind to a DNA scaffold to form the DNA-nanostructure, wherein the sequence of staples configured to bind to the DNA scaffold forms at least one of: the 2D DNA-nanostructure, and the 3D DNA-nanostructure.

7. The probe according to any of the preceding and with features of claim 5, wherein the target binding sequence comprises a complementarity to the subsequence of the RNA to be detected of at least 75%, preferably at least 85%, more preferably at least 95%.

8. A composition for RNA FISH comprising a set of probes for RNA FISH, wherein the set of probes comprises at least 1 probe for RNA FISH, preferably at least 5 probes for RNA FISH, more preferably at least 10 probes for RNA FISH, wherein the set of probes comprises at least one probe according to any of claims 1 to 7.

9. The composition according to the preceding claim, wherein the set of probes comprises at least one probe binding to a sub-sequence of a target RNA sequence to result in at least one bound probe, wherein the at least one bound probe is detectable using fluorescence microscopy.

10. A method for designing a probe for RNA FISH for analyzing a sample, wherein the method comprises providing a probe for RNA FISH, wherein the probe is according to any of claims 1 to 7, wherein the method comprises detecting at least one target molecule in situ in the sample using the probe.

11. The method according to the claim, wherein the method comprises at least one of:
combining at least one flurophore molecule comprising a single color with one marker molecule linked to the probe with the target molecule, wherein the single color comprises at least two intensity levels of occurrence, wherein each of the at least two intensity levels of occurrence directly correlates to the number of fluorophore molecules per marker molecule; and
combining at least one fluorophore molecule comprising at least one color with one marker molecule linked to the probe with the target molecule, wherein the at least one color comprises at least two different types of fluorophores, wherein each of the at least one color comprises at least two intensity levels of occurrence, wherein each of the at least two intensity levels of occurrence directly correlates to the number of fluorophore molecules per marker molecule.

12. The method according to any of claims 10 to 11, wherein the at least one target molecule is bound to the probe, wherein the method comprises detecting the least one target molecule to probe, wherein the method comprises imaging the sample, collecting at least one z-slice image, removing a background from the at least one z-slice image, identifying a number of individual, dot-like signals in a given channel, and counting the number of individual, dot-like signals identified in the given channel, wherein the at least one z-slice image comprises a plurality of images in a z distance fitting a resolution limit of a microscope.

13. The method according to any of claims 10 to 12, wherein designing the probe comprises comprising
defining via search in an mRNA repository a coding sequence (CDS) of a RNA of interest comprising a target RNA sequence,
folding the target RNA sequence under hybridization conditions,
analyzing the RNA of interest for an open loop structure when folding the target RNA sequence under hybridization conditions,
determining a probe length of n nucleotides,
calculating a score for each n nucleotide sub-sequence, and
estimating an average probability of a long sub-sequence to be an open loop, wherein the calculating step is performed iteratively in the CDS in single nucleotide increments for each stretch of n nucleotides,
wherein the method comprises
selecting between 5 and 10 sub-sequences with the highest scores as probe targets,
expanding the selected sub-sequence by 10 to 200 nt on each side to output stretched nucleotides,
computationally probing the stretched nucleotides for matches of 22 nt to 50 nt probes, preferably at least 22 nt and less than 41 nt, more preferably less than 30 nt, and
selecting matching sub-sequences based on thermodynamics comprising low likelihood of structure under buffer conditions of Na⁺ 300 mM and Mg²⁺ 11 mM.

14. A kit for use in a molecular analysis, the comprising a probe according to any of claims 1 to 7, or a composition according to any of claims 8 or 9, and at least one buffer for hybridization comprising at least one of: fixation buffer component, wash buffer, and hybridization buffer.

15. The kit according to the preceding claim, wherein the
probe is at least one of: a solution, and freeze-dried;
composition comprises at least one probe according to any of the preceding probe claims, wherein the at least one probe is at least one of: in a solution and freeze-dried.
